# EUROPEAN PATENT APPLICATION

(11) **EP 0 995 798 A1**
(43) Date of publication of application: **26.04.2000**
(21) Application number: 98120085.0
(22) Date of filing: 24.10.1998
(51) Int. Cl.: C12N 15/12, C07K 14/47, C07K 16/18

(54) **Transcription factor of MHC class II genes, substances capable of inhibiting this new transcription factor and medical uses of these substances**

(71) Applicant: NOVIMMUNE SA, 1207 Geneva (CH)
(72) Inventor: Masternak, Krzysztof, 1205 Genève (CH); Reith, Walter, 1227 Carouge (CH); Mach, Bernard, 1292 Pregny-Chambesy (CH)
(74) Representative: Almond-Martin, Carol

(57) **Abstract**

The present invention relates to a novel trancription factor of MHC class II genes and its derivatives, inhibitors of this transcription factor capable of down-regulating the expression of MHC class II molecules and medical uses of these inhibitors.

SEQUENCE LISTING

<110> MEDIGEN SA c/o LENZ AND STAEHELIN

<120> NEW TRANSCRIPTION FACTOR OF MHC CLASS II GENES,
      SUBSTANCES CAPABLE OF INHIBITING THIS NEW TRANSCRIPTION
      FACTOR AND MEDICAL USES OF THESE SUBSTANCES

<130> B3991GD/LL

<140> 98120085.0
<141> 1998-10-23

<160> 19

<170> PatentIn Ver. 2.1

<210> 1
<211> 40
<212> DNA
<213> Artificial Sequence

<220>
<223> Description of Artificial Sequence:primer

<400> 1
ccgtacgcgt ctagaccatg gagcttaccc agcctgcaga                                40

<210> 2
<211> 31
<212> DNA
<213> Artificial Sequence

<220>
<223> Description of Artificial Sequence:primer

<400> 2
ttcgaattct cgagtgtctg agtccccggc a                                         31

<210> 3
<211> 37
<212> DNA
<213> Artificial Sequence

<220>
<223> Description of Artificial Sequence:primer

<400> 3
ccgtacgcgt ctagaccatg gagcccactc aggttgc                                37

<210> 4
<211> 32
<212> DNA
<213> Artificial Sequence

<220>
<223> Description of Artificial Sequence:primer

<400> 4
ttcgaattct cgagtgcctg ggttccagca gg                                     32

<210> 5
<211> 30
<212> DNA
<213> Artificial Sequence

<220>
<223> Description of Artificial Sequence:primer

<400> 5
ccagctctag actccaccac tctcaccaac                                        30

<210> 6
<211> 30
<212> DNA
<213> Artificial Sequence

<220>
<223> Description of Artificial Sequence:primer

<400> 6
ccttcgaatt ctcgctcttt tgccaggatg                                        30

<210> 7
<211> 30
<212> DNA
<213> Artificial Sequence

<220>
<223> Description of Artificial Sequence:primer

<400> 7
ggttctctag attggcagca ctggggatag                                        30

<210> 8
<211> 30
<212> DNA
<213> Artificial Sequence

<220>
<223> Description of Artificial Sequence:primer

<400> 8
gctacgaatt ccagcagaca cagccaaaac                                        30

<210> 9
<211> 69
<212> DNA
<213> Artificial Sequence

<220>
<223> Description of Artificial Sequence:primer

<400> 9
ccgtacgcgt ctagaatgga ttacaaagac gatgacgata agatggagct tacccagcct 60

gcagaagac                                                               69

<210> 10
<211> 1345
<212> DNA
<213> Homo sapiens

<220>
<221> CDS
<222> (418)..(1200)

<400> 10
acgcagggaa ggaggcacac ccgggggtgg cgcagtgagg agggggcgcg acggccagga 60
ggctggtgga gcgacaccca ggcaggagag ggggaagaac tctctccctt tctgaacccc 120
cttttccttg agagacgagt tgggggagtc ctccacgcat tacccactcg ggccgcaaaa 180
actcccttct ttagccctct gcccccgccc ttgcttataa gcctttgaga ccgcagaagg 240
gaccttgttg tggaacggga cggccaagag gaagccagat cgctgagggt ccggtctcca 300
gtttgcctcc tgctatatcc attggaagag aaaagtttgt gacttgggcc cccaagtttt 360
gagagaactg ggctttcggc gcggggggac agaggaggct cgtggggagc tttcccc 417
atg gag ctt acc cag cct gca gaa gac ctc atc cag acc cag cag acc 465
cct gcc tca gaa ctt ggg gac cct gaa gac ccc gga gag gag gct gca 513
gat ggc tca gac act gtg gtc ctc agt ctc ttt ccc tgc acc cct gag 561
cct gtg aat cct gaa ccg gat gcc agt gtt tcc tct cca cag gca ggc 609
agc tcc ctg aag cac tcc acc act ctc acc aac cgg cag cga ggg aac 657
gag gtg tca gct ctg ccg gcc acc cta gac tcc ctg tcc atc cac cag 705
ctc gca gca cag ggg gag ctg gac cag ctg aag gag cat ttg cgg aaa 753
ggt gac aac ctc gtc aac aag cca gac gag cgc ggc ttc acc ccc ctc 801
atc tgg gcc tcc gcc ttt gga gag att gag acc gtt cgc ttc ctg ctg 849
gag tgg ggt gcc gac ccc cac atc ctg gca aaa gag cga gag agc gcc 897
ctg tcg ctg gcc agc aca ggc ggc tac aca gac att gtg ggg ctg ctg 945
ctg gag cgt gac gtg gac atc aac atc tat gat tgg aat gga ggg acg 993
cca ctg ctg tac gct gtg cgc ggg aac cac gtg aaa tgc gtt gag gcc 1041
ttg ctg gcc cga ggc gct gac ctc acc acc gaa gcc gac tct ggc tac 1089
acc ccg atg gac ctt gcc gtg gcc ctg gga tac cgg aaa gtg caa cag 1137
gtg atc gag aac cac atc ctc aag ctc ttc cag agc aac ctg gtg ccc 1185
gct gac cct gag tga aggccgcctg ccggggactc agacactcag ggaacaaaat 1240
ggtcagccag agctggggaa acccagaact gacttcaaag gcagcttctg gacaggtggt 1300
gggaggggac ccttcccaag aggaaccaat aaaccttctg tgcag 1345

<210> 11
<211> 260
<212> PRT
<213> Homo sapiens

<400> 11

<210> 12
<211> 260
<212> PRT
<213> Homo sapiens

<400> 12

<210> 13
<211> 269
<212> PRT
<213> Murinae gen. sp.

<400> 13
Met Glu Pro Thr Gln Val Ala Glu Asn Leu Val Pro Asn Gln Gln Pro
  1               5                  10                  15

Pro Val Pro Asp Leu Glu Asp Pro Glu Asp Thr Arg Asp Glu Ser Pro
             20                  25                  30

Glu Asn Ser Asp Thr Val Val Leu Ser Leu Phe Pro Cys Thr Pro Asp
         35                  40                  45

Ala Val Asn Pro Glu Ala Asp Ala Ser Ala Ser Ser Leu Gln Gly Ser
     50                  55                  60

Phe Leu Lys His Ser Thr Thr Leu Thr Asn Arg Gln Arg Gly Asn Glu
 65                  70                  75                  80

Val Ser Ala Leu Pro Ala Thr Leu Asp Ser Leu Ser Ile His Gln Leu
                 85                  90                  95

Ala Ala Gln Gly Glu Leu Ser Gln Leu Lys Asp His Leu Arg Lys Gly
            100                 105                 110

Ala Cys Pro Ala Cys Thr Cys Leu Ser Gly Asn Asn Leu Ile Asn Lys
        115                 120                 125

Pro Asp Glu Arg Gly Phe Thr Pro Leu Ile Trp Ala Ser Ala Phe Gly
    130                 135                 140

Glu Ile Glu Thr Val Arg Phe Leu Leu Asp Trp Gly Ala Asp Pro His
145                 150                 155                 160

Ile Leu Ala Lys Glu Arg Glu Ser Ala Leu Ser Leu Ala Ser Met Gly
                165                 170                 175

Gly Tyr Thr Asp Ile Val Arg Leu Leu Leu Asp Arg Asp Val Asp Ile
            180                 185                 190

Asn Ile Tyr Asp Trp Asn Gly Gly Thr Pro Leu Leu Tyr Ala Val Arg
        195                 200                 205

Gly Asn His Val Lys Cys Val Glu Ala Leu Leu Ala Arg Gly Ala Asp
    210                 215                 220

Leu Thr Thr Glu Ala Asp Ser Gly Tyr Thr Pro Met Asp Leu Ala Val
225                 230                 235                 240

Ala Leu Gly Tyr Arg Lys Val Gln Gln Val Met Glu Ser His Ile Leu
                245                 250                 255

Arg Leu Phe Gln Ser Thr Leu Gly Pro Val Asp Pro Glu
            260                 265

<210> 14
<211> 111
<212> DNA
<213> Homo sapiens

<220>
<221> CDS
<222> (1)..(111)

<400> 14
acc cta gac tgg tgc cga ccc cca cat cct ggc aaa aga gcg aga gag     48
Thr Leu Asp Trp Cys Arg Pro Pro His Pro Gly Lys Arg Ala Arg Glu
  1               5                  10                  15

cgc cct gtc gct ggc cag cac agg cgg cta cac aga cat tgt ggg gct     96
Arg Pro Val Ala Gly Gln His Arg Arg Leu His Arg His Cys Gly Ala
             20                  25                  30

gct gct gga gcg tga                                                 111
Ala Ala Gly Ala
         35

<210> 15
<211> 37
<212> PRT
<213> Homo sapiens

<400> 15
Thr Leu Asp Trp Cys Arg Pro Pro His Pro Gly Lys Arg Ala Arg Glu
1               5                   10                  15

Arg Pro Val Ala Gly Gln His Arg Arg Leu His Arg His Cys Gly Ala
            20                  25                  30

Ala Ala Gly Ala
        35

<210> 16
<211> 42
<212> DNA
<213> Homo sapiens

<220>
<221> exon
<222> (31)..(42)

<400> 16
ctggtggtat tgcccgcctc ctcctgccag gtg aca acc tcg                42

<210> 17
<211> 74
<212> DNA
<213> Homo sapiens

<220>
<221> exon
<222> (1)..(27)

<400> 17
gag acc gtt cgc ttc ctg ctg gag tgg gtgcgtccca gcccagctgg        47

gcagctgggg ggttcccggg ggcctta                                    74

<210> 18
<211> 220
<212> PRT
<213> Homo sapiens

<400> 18
Asn Ala Phe Asn Val Phe Thr Phe Val Phe His Leu Ala Glu Cys Asn
1               5                   10                  15

Ile His Thr Ser Pro Ser Pro Gly Ile Gln Val Arg His Val Xaa Thr
            20                  25                  30

Pro Ser Thr Thr Lys His Phe Ser Pro Ile Lys Gln Ser Thr Thr Leu
        35                  40                  45

Thr Asn Lys His Arg Gly Asn Glu Val Ser Thr Thr Pro Leu Leu Ala
    50                  55                  60

Asn Ser Leu Ser Val His Gln Leu Ala Ala Gln Gly Glu Met Leu Tyr
65                  70                  75                  80

Leu Ala Thr Arg Ile Glu Gln Glu Asn Val Ile Asn His Thr Asp Glu
                85                  90                  95

Glu Gly Phe Thr Pro Leu Met Trp Ala Ala Ala His Gly Gln Ile Ala
            100                 105                 110

Val Val Glu Phe Leu Leu Gln Asn Gly Ala Asp Pro Gln Leu Leu Gly
        115                 120                 125

Lys Gly Arg Glu Ser Ala Leu Ser Leu Ala Cys Ser Lys Gly Tyr Thr
    130                 135                 140

Asp Ile Val Xaa Met Leu Leu Asp Cys Gly Val Asp Val Asn Xaa Tyr
145                 150                 155                 160

Asp Trp Asn Gly Gly Thr Pro Leu Leu Tyr Ala Val His Gly Asn His
                165                 170                 175

Val Lys Cys Val Lys Met Leu Leu Glu Ser Gly Ala Asp Pro Thr Ile
            180                 185                 190

Glu Thr Asp Ser Gly Tyr Asn Ser Met Asp Leu Ala Val Ala Leu Gly
        195                 200                 205

Ile Glu Val Phe Asn Arg Leu Leu Ser His Ile Cys
    210                 215                 220

<210> 19
<211> 218
<212> PRT
<213> Murinae gen. sp.

<400> 19
Ala Ser Val Leu Phe Lys Ala Glu Cys Asn Ile His Thr Ser Pro Ser
1               5                   10                  15

Pro Gly Ile Gln Val Arg His Val Tyr Thr Pro Ser Thr Thr Lys His
            20                  25                  30

Phe Ser Pro Ile Lys Gln Ser Thr Thr Leu Thr Asn Lys His Arg Gly
        35                  40                  45

Asn Glu Val Ser Thr Thr Pro Leu Leu Ala Asn Ser Leu Ser Ala His
    50                  55                  60

Gln Leu Ala Ala Gln Gly Glu Met Leu Tyr Leu Ala Thr Arg Ile Glu
65                  70                  75                  80

Gln Glu Asn Val Ile Asn His Thr Asp Glu Glu Gly Phe Thr Pro Leu
                85                  90                  95

Met Trp Ala Ala Ala His Gly Gln Ile Ala Val Val Glu Phe Leu Leu
            100                 105                 110

Gln Asn Gly Ala Asp Pro Gln Leu Leu Gly Lys Gly Arg Glu Ser Ala
        115                 120                 125

Leu Ser Leu Ala Cys Ser Lys Gly Tyr Thr Asp Ile Val Lys Met Leu
    130                 135                 140

Leu Asp Cys Gly Val Asp Val Asn Glu Tyr Asp Trp Asn Gly Gly Thr
145                 150                 155                 160

Pro Leu Leu Tyr Ala Gly His Gly Asn His Val Lys Cys Val Lys Met
                165                 170                 175

Leu Leu Glu Asn Gly Ala Asp Pro Thr Ile Glu Thr Asp Ser Gly Tyr
            180                 185                 190

Asn Ser Met Asp Leu Ala Val Ala Leu Gly Ile Glu Gly Cys Ser Asp
        195                 200                 205

Tyr Met Leu Val Thr Asp Val Phe Arg Ile
    210                 215

## Description

The present invention relates to a novel trancription factor of MHC class II genes and its derivatives, inhibitors of this transcription factor capable of down-regulating the expression of MHC class II molecules and medical uses of these inhibitors.

MHC class II molecules, for example HLA-DR, HLA-DQ and HLA-DP in humans are transmembrane heterodimers that are essential for antigen presentation and activation of T lymphocytes. They are encoded by a multi-gene family and are highly regulated in their expression.

Abnormal or aberrant expression of MHC class II genes leads to an aberrant T cell activation, which leads to an abnormal immune response.

Such abnormal immune response is a cause of inflammation events, autoimmune diseases or rejections of transplanted organs.

There is an important need to downregulate the expression of MHC class II molecules in order to treat or prevent the above cited clinical events.

A powerful means of obtaining MHC class II downregulation or immunosuppression consists in intervening on the transcription (transcriptional intervention) of the MHC class II genes.

Transcriptional intervention can be achieved by acting on the transcription factors which are involved in the transcription of MHC class II genes.

The transcription factor which is the target of the transcriptional intervention has to be essential and specific for the expression of the genes that one wants to inhibit.

Such essential and specific transcription factors are however rare.

Therefore, there is a great need to identify such a transcription factor.

The invention relates to such a new transcription factor called RFX-ANK which is a subunit of the heterotrimeric transcription complex called RFX that binds to the conserved X box motif of all MHC II promoters.

Much of what we know today about this important transcriptional factor called RFXANK results from the study of a rare genetic disease related to MHC class II deficiency.

MHC class II deficiency is an unusual autosomal recessive disease in which the genes implicated in the phenotype (MHC-II genes) are in fact intact. Instead, the original defect results from mutations in several different trans-acting regulatory genes responsible for the regulation and expression of MHC-II genes^{1,2}.

In MHC-II deficiency, there is a complete loss of expression of all MHC-II genes, which leads to a form of severe primary immunodeficiency^{1,2}. Patients have repeated infections and frequently die before the age of 10. All clinical manifestations and phenotypic features of these patients can be attributed to the defect in the expression of MHC-II genes^{3,4}. Because of the key role of the tight and complex regulation of MHC-II genes in the control of the immune response, the recognition of MHC-II deficiency as a disease of gene regulation has led to a wide interest in the search for the regulatory factors that are involved. These factors are important elements controlling the development, homeostasis and effector functions of the immune system.

Although clinically homogeneous, the disease is genetically heterogeneous. There are four distinct genetic complementation groups (A-D, refs.5-7), which reflects the existence of four essential regulators of MHC-II expression². The regulatory genes that are defective in complementation groups A (MHC2TA), C (RFX5) and D (RFXAP) have been identified⁸⁻¹⁰. CIITA (MHC2TA gene product) is a non DNA binding co-activator, whose expression controls the cell type specificity and inducibility of MHC-II genes^{8,11,12}. RFX5 and RFXAP are two subunits of RFX, a multi-protein complex that binds to the conserved X box motif of all MHC-II promoters^{13,14}. Mutations in either RFX5 or RFXAP abolish binding of RFX and transcription of MHC-II genes^{9,10,15}.

The molecular defect responsible for complementation group B, although it accounts for the great majority of MHC-II deficiency patients, has remained elusive. Like complementation groups C and D, group B is characterized by a specific lack of binding of the RFX complex to the X box of MHC-II promoters^{2,13,14}, suggesting a defect in an additional subunit of RFX. However, neither complementation of defective cells, an approach that had led to the cloning of MHC2TA (ref.8) and RFX5 (ref.9) genes, nor classical affinity purification of the RFX complex, which had allowed the cloning of RFXAP (ref.10), have permitted us, or other laboratories, to identify the gene that is affected in complementation group B.

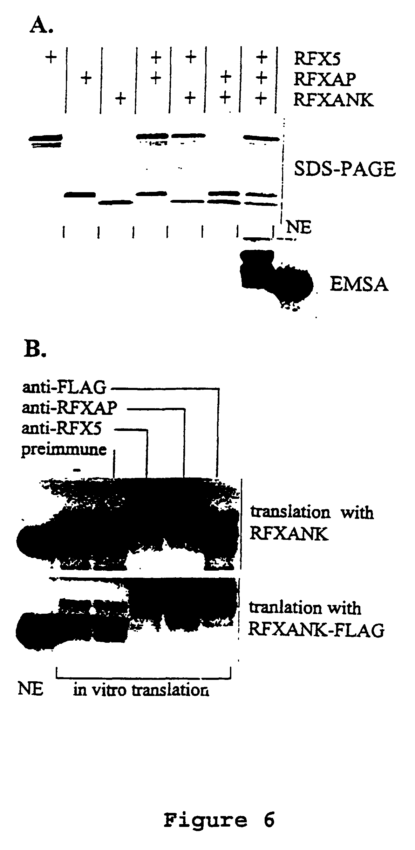

The fact that the great majority of all MHC-II deficiency patients belong to group B had highlighted this group as a challenge. This remained the case after the cloning of the three regulatory genes affected in groups A, C and D (refs.8-10). We and others have attempted to solve group B by a variety of strategies. These have included functional complementation assays similar to those that had led to the discovery of CIITA (ref.8) and RFX5 (ref.9), classical multistep purification, as was used in the identification of RFXAP (ref.10), mutagenesis by retroviral insertion, two hybrid selection systems in yeast and genetic localization by linkage studies. The approach that finally turned out to be successful is an efficient single step DNA affinity purification procedure that exploits the remarkable stability of a large multi-protein complex formed by RFX, X2BP, and NF-Y, which bind cooperatively to a longer segment of MHC-II promoters (the X-X2-Y box region)^{18,19,23} (see Fig. 6). The very high stability of this large complex represents an obvious advantage in terms of the yield and enrichment obtained in the purification procedure. Another major advantage is that it allows the selection of those factors that are part of a physiologically relevant multi-protein―DNA complex, at the expense of numerous other proteins capable of binding individually to the isolated X, X2, and Y motifs. Thus, in addition to RFX purification, it allows the co-purification of the biologically relevant X2 and Y box-binding factors.

Thus, here we report the isolation of a novel subunit of the RFX complex by an efficient single-step DNA-affinity purification approach. This procedure takes advantage of a strong co-operative protein-protein interaction that results in the highly stable binding of three distinct multi-protein transcription factors ― RFX, X2BP (ref.16) and NF-Y (ref.17) ― to the X-X2-Y region of MHC-II promoters^{10,18,19}. The higher order RFX―X2BP―NF-Y complex formed on DNA contains the physiologically relevant proteins involved in the activation of MHC-II promoters. With the use of this new affinity purification procedure, the novel component of the RFX complex called RFXANK was identified and the corresponding gene was isolated.

This gene is capable of fully correcting the MHC-II expression defect of cell lines from several patients in complementation group B and it was indeed found to be mutated in these patients. The amino acid sequence revealed a series of ankyrin repeats, a well defined protein-protein interaction motif^{20,21}, and this novel factor was therefore called RFXANK. We also demonstrate here that RFXANK is essential for binding of the RFX complex to the X box of MHC -II promoters.

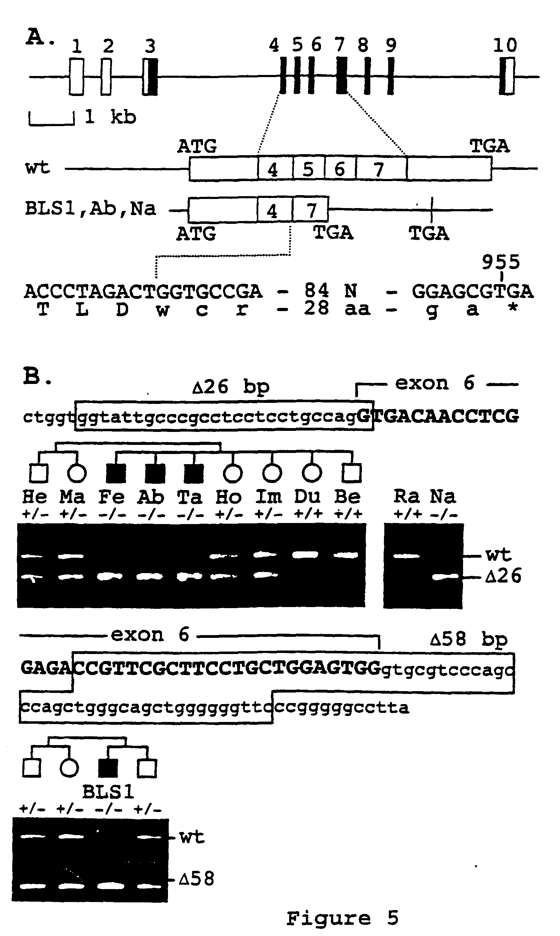

RFXANK is shown here to have a dramatic effect on the binding of the RFX complex to the X box motif. RFX5, which possesses a characteristic DNA-binding domain (the RFX DBD motif²⁴), cannot bind to its natural DNA target, either alone⁹, or in the presence of RFXAP (ref.10). The X box-binding activity is restored, however, when RFX5 and RFXAP are co-translated together with RFXANK, demonstrating that the presence of each of the three subunits is required (Fig. 5). The existence of ankyrin repeats within RFXANK suggests that this motif mediates interactions between the different subunits of the RFX complex. Ankyrin repeats are protein-protein interaction motifs known to be implicated in a wide range of biologically important regulatory events, such as the binding of the p53 binding protein (53BP2) to its target³⁰, of IkB to NF-kB (refs.31,32) and of INK4 kinase inhibitors to CDK4/6 (ref.33). However, ankyrin repeats have been described only rarely in transcription factors. An example of particular interest is GABP, a heterodimeric transcription factor composed of a DNA-binding a subunit and a b subunit containing ankyrin repeats^{28,29} . Interestingly, the binding of GABPb to GABPa ― which is mediated by the ankyrin region of GABPb ― greatly enhances the affinity of GABPa to its DNA target^{34,35}. By analogy, we propose that interactions between RFXANK and RFX5, in the presence of RFXAP, induce structural alterations that allow the DNA binding domain of RFX5 to bind the X box of MHC-II promoters. Alternatively, the ankyrin repeats of RFXANK may be involved in other functionally essential interactions, such as the cooperative interactions that stabilize the higher order RFX―X2BP―NF-Y complex or direct contacts between RFX and the co-activator CIITA.

The human RFXANK gene was identified in genomic sequences available in GenBank. This allowed us to establish rapidly the entire intron-exon organization of the RFXANK gene (see Fig. 5). Furthermore, it allowed us to confirm and extend our initial chromosomal mapping to 19p of the gene involved in group B of MHC-II deficiency. This localization was obtained from linkage studies with a small number of informative families. The RFXANK gene is located on human chromosome 19, at 19p12, between markers D19S566 and D19S435 (see GenBank accession numbers AD000812 and AC003110). The availability of the RFXANK gene will make possible an analysis of potential polymorphism. We suggest that the RFXANK gene, like the three other transactivator genes that control MHC-II expression, should be examined closely for possible polymorphic diversity, either in coding or control regions. This may be relevant to various forms of immunopathology.

RFX5 and RFXAP are expressed constitutively in all cell types tested and the corresponding genes are not known to be regulated. It is likely that expression of the RFXANK gene exhibits the same ubiquitous pattern since the corresponding ESTs (over 80 different clones) have been isolated from a wide variety of different tissues. The gene encoding CIITA (MHC2TA), on the other hand, is extremely tightly regulated, with a constitutive expression that is restricted to professional antigen presenting cells. It is the expression of CIITA, either constitutive in certain specialized cells or inducible in others, that determines activation of MHC-II promoters^{11,12}. Thus, two distinct regulatory components, the ubiquitously expressed RFX complex and the highly regulated transactivator CIITA, must act in concert.

Several features make MHC-II deficiency unique among diseases of gene regulation. First, patients from the different genetic complementation groups have indistinguishable clinical features²⁻⁴. Thus, defects in four distinct and unrelated genes (MHC2TA, RFX5, RFXAP and RFXANK) all result in a single disease having a remarkably homogeneous clinical and biological phenotype. Second, the factors involved in this disease exhibit a very tight control over MHC-II gene expression. This control cannot be compensated for by other redundant regulatory mechanisms. In contrast, defects in many other regulatory genes such as Oct2, Obf1 or MyoD1 frequently reveal the existence of alternative pathways of activation of the specific genes that are under their control. Third and most important, no other genes, except for the MHC-II related HLA-DM and Ii genes, are known to be affected significantly in MHC-II deficiency. All observed symptoms and biochemical features of the disease can be attributed to a single defect, namely a general lack of both constitutive and inducible expression of MHC-II genes in all cell types²⁻⁴. This indicates that all four trans-activating factors (CIITA, RFX5, RFXAP and RFXANK) are dedicated to the control of MHC-II genes. This restriction in specificity is also uncommon.

Indeed, genetic defects in many transcription factors, in particular in those affected in human diseases, result in pleiotropic effects almost always involving complex developmental disorders (for review see 36). This is because the majority of regulatory genes affected in human diseases ― in contrast to the four transactivators defective in MHC-II deficiency ― are implicated in the control of multiple different target promoters and genes. In the case of the three subunits of RFX, the restricted specificity contrasts with their ubiquitous pattern of expression.

The discovery of RFXANK as the gene affected in complementation group B of MHC-II deficiency has allowed us to complete the elucidation of the molecular and genetic basis of this disease. Two features make this disease of gene regulation highly unusual: First, defects in any one of four entirely different transcriptional transactivators lead to the very same clinical and phenotypic manifestations. Second, each of these four MHC-II transactivators is not only essential for the control of MHC-II gene expression but is also dedicated to the control of these genes, without playing any major role in the transcriptional control of other genes.

It is known from the phenotype of patients of complementation group B and of cell lines from such patients, that RFX-ANK factor is indeed both essential for the activity of MHC class II genes and specific in its effect on these genes.

Thus, the new transcription factor of the invention has two essential and unusual characteristics that makes it suitable as a target for transcriptional intervention.

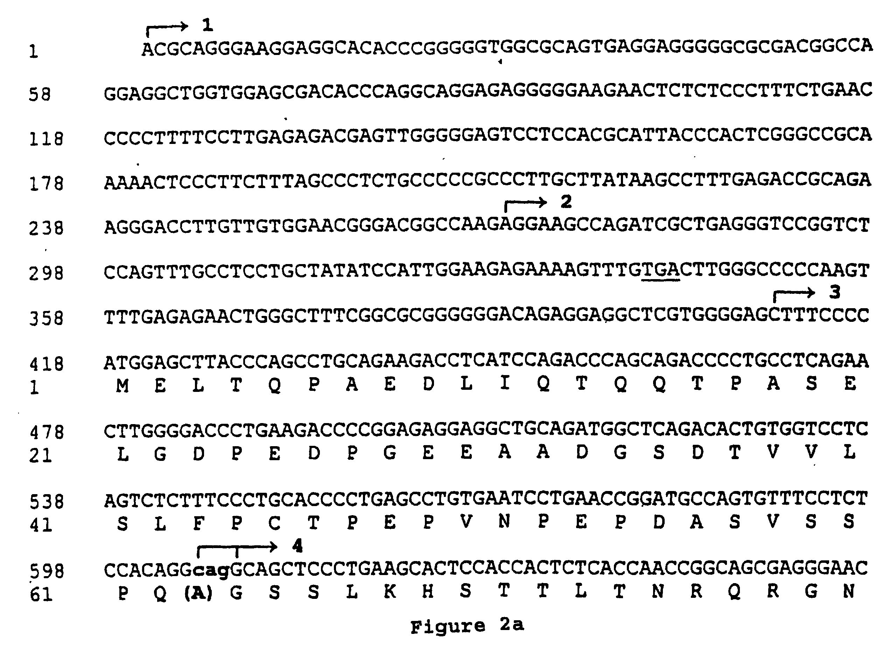

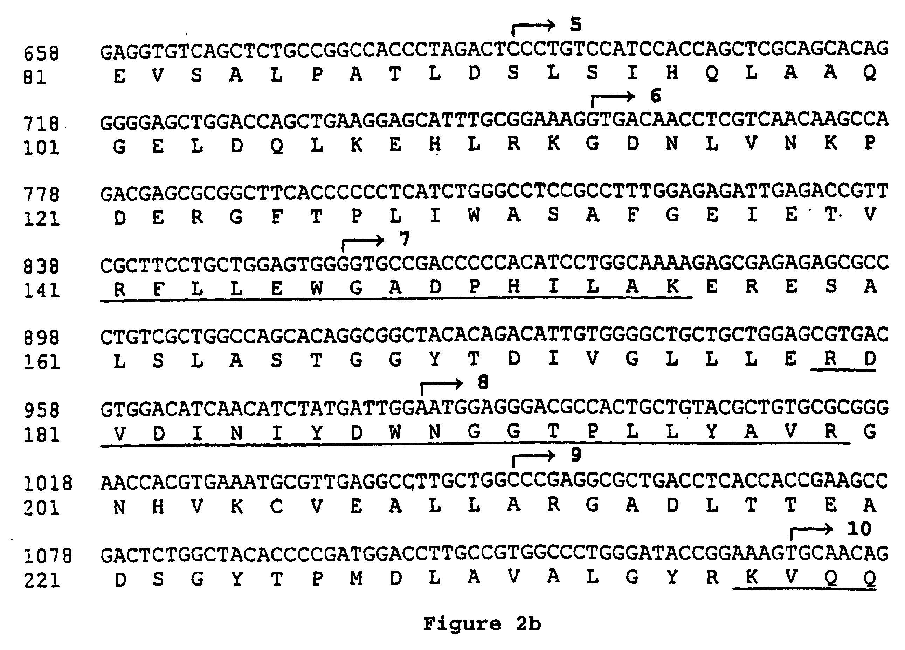

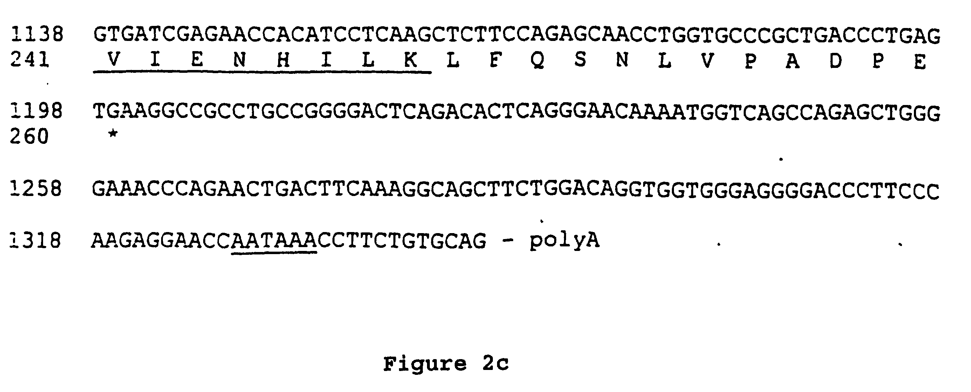

The invention relates to a protein or a peptide capable of restoring the MHC II expression in cells from MHC II defiency patients in complementation group B and comprising all or part of the amino acid sequence shown in figure 2.

The cells from MHC II deficiency patients in complementation group B may be chosen among BLS1 cell lines (reference 6), Na cell lines (reference 40) or Ab cell lines (reference 14).

The MHC II molecules which are restored by a protein or a peptide of the invention can be chosen among the HLA-DR, HLA-DP or HLA-DQ molecules.

The invention relates to a protein or a peptide comprising the amino acid sequence shown in figure 2 and part thereof, or an amino acid sequence having at least 80 % and preferably at least 90 % homology with the illustrated sequences and part thereof. The homologous sequences and parts thereof are referred to as "derivatives".

Part of a protein or a peptide may be fragments of at least 6 amino acids, preferably at least 20 amino acids.

The amino acid sequence shown in figure 2 is the human sequence of RFX-ANK. Derivatives of the figure 2 sequence may be agonists or antagonists of the RFX-ANK function as defined below.

In a further embodiment, the invention relates to a protein or a peptide comprising the amino acid sequence of RFX-ANK of other species than human and part thereof, especially pig and to the amino acid sequence having at least 80 % and preferably at least 90 % homology with these amino acid sequences and part thereof.

The amino acid sequence of RFX-ANK of other species can be obtained by standard methods like cross hybridization at low stringency (see Maniatis).

The invention also relates to an antibody capable of specifically recognising a protein or a peptide of the invention.

Such antibody can be obtained by standard methods. In vitro methods are for example ELISA assays. In vivo methods consist for example in administering to an organism a protein or a peptide of the invention in order to produce antibodies specific of the administered protein or peptide.

In vivo and in vitro methods may of course be used in a complementary way to obtain antibody.

Antibodies of the invention may be monoclonal. Monoclonal antibody can for example be produced by the technique of hybridoma.

Antibody of the invention may be single chain antibody. Techniques to generate single chain antibody are well known.

The invention also relates to a nucleic acid molecule (RNA, DNA or cDNA) encoding a protein, a peptide or an antibody of the invention.

The nucleic acid molecule of the invention may comprise all or part of the nucleotide sequence illustrated in figure 2 (GenBank Accession Number : Human RFXANK cDNA AF094760).

A part of a nucleic acid molecule or of a nucleotide sequence corresponds to at least 18 nucleotides, and preferentially at least 60 nucleotides.

The invention relates also to nucleic acid molecule capable of hybridizing in stringent condition with the nucleic acid molecule of the invention.

Typical stringent conditions are those where the combination of temperature and salt concentration chosen to be approximately 12-20°C below the Tm (melting temperature) of the hybrid under study.

In a further embodiment, the invention relates to a nucleic acid molecule comprising the nucleotide sequence illustrated in figure 2 to a nucleotide sequence exhibiting at least 90 % identity with said nucleotide sequence or to a part of said nucleotide sequence.

In another embodiment, the invention relates to the nucleotide sequence of the RFX-ANK gene of other species than human (especially pigs) to the nucleotide sequence having at least 60 % or 70% identity with said nucleotide sequence and to a part of said nucleotide sequences.

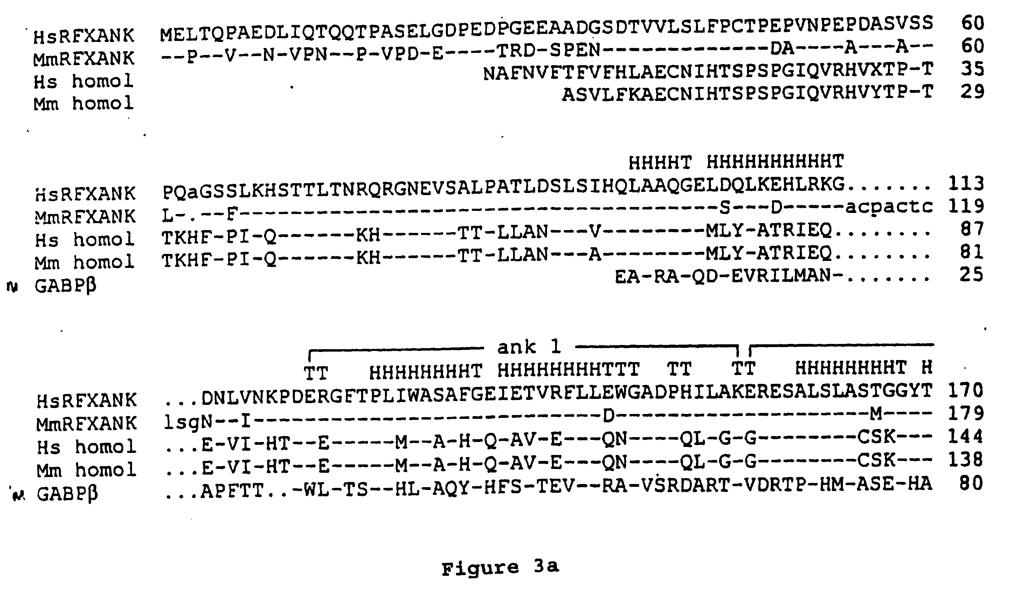

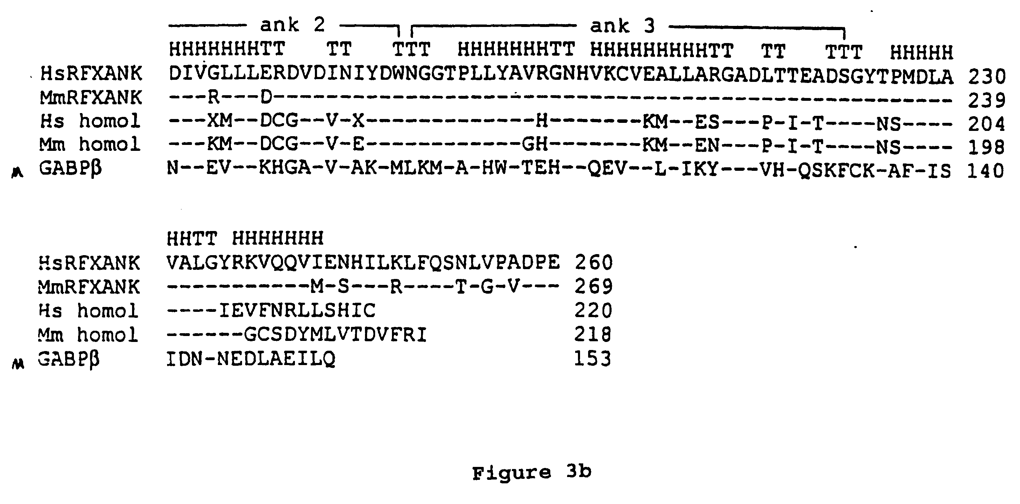

The invention relates particularly to the mouse RFXANK nucleotide sequence (GenBank Accession Number : Mouse RFXANK cDNA AF094761-see figure 3), to the nucleotide sequence having at least 60% or 70 % identity with these nucleotide sequence and to a part of said nucleotide sequences.

In another embodiment, the invention relates to a nucleic acid molecule comprising a sequence complementary to the nucleic acid molecule of the invention.

In a further embodiment, the invention relates to an anti-sense molecule or a ribozyme comprising a nucleic acid molecule as recited by the preceeding paragraph.

The nucleic acid molecule of the invention may have at least 20 nucleotides and preferably at least 40.

The invention relates also to a vector comprising a nucleic acid molecule of the invention or being able to express an anti-sense molecule or a ribozyme of the invention.

A peptide, a protein or a nucleic acid molecule of the invention is called a transcription factor of the invention.

After the transcriptional intervention target (transcriptional factor of the invention) has been identified, the transcriptional intervention then requires the identification of inhibitors or inhibitory molecules (by biochemical in vitro screening and/or by cell based screening) followed by tests of putative inhibitors in animal models.

〈〈 Inhibitory molecules 〉〉 or 〈〈 inhibitors 〉〉 of a molecule are substances which have the capacity to inhibit a function, an activity or the synthesis of a molecule of interest.

The molecule of interest may be a nucleic acid molecule or a peptide or a protein.

The capacity to inhibit may be the partial or total capacity to block, repress, suppress, stop, abolish, compete with or downregulate, the function, or the synthesis of the molecule of interest.

The molecule of interest of the present invention is a protein, a peptide or a nucleic acid molecule of the invention encoding said protein or peptide. The molecule is called a transcription factor of the invention. The transcription factors may be RFX-ANK as shown in figure 2 or derivatives thereof as described earlier.

The function of a transcription factor of the invention, particularly the transcription factor RFX-ANK and its functional derivatives can be defined as the capacity to enable the functional transcription of MHC class II genes, via the RFX complex, and consequently the expression of MHC class II gene products.

This function can be recognised as the capacity to correct the MHC II expression defect of cell lines from patients in complementation group B. The function of the molecules of the invention is achieved globally by a series of sequential steps involved. Thus, each of these steps can be considered, in the context of the invention, as being the direct or indirect function of the molecules of interest of the invention.

Consequently, the function or activity of the molecule of interest of the invention (transcription factor of the invention) signifies the capacity to allow the expression of MHC class II molecules, to allow the transcription of an MHC class II gene, to allow the expression or the translation of an MHC class II protein or peptide, to allow the formation of the RFX complex, to allow the binding of the RFX complex to the X box motif, to allow the interaction between the RFX complex and at least one of the transcription factors X2BP, NF-Y or CIITA, to allow a cooperative interaction that stabilizes the higher order RFX-X2BP-NF-Y complex, to direct contacts between RFX and the co-activator CIITA, to allow binding of RFX5 to the X box, or to correct the MHC II expression defect of cell lines from patients in complementation group B.

The synthesis of the molecule of interest of the invention (transcription factor of the invention) may be the transcription and/or translation of the nucleic acid molecule of the invention into the protein or peptide of the invention encoded by said nucleic acid molecule.

Due to the fact that the transcription factor of the invention does not play any major role in the transcriptional control of other genes than MHC class II genes, inhibitors of said transcription factor of the invention are devoid of other undesirable inhibitory effects.

Inhibitors of the invention have a very specific action limited to MHC class II genes because their target, ie transcription factor of the invention has specificity restricted to promoters of MHC class II genes.

In fact, a transcription factor of the invention does not appear to be implicated in the control of other target promoters and genes.

Furthermore, inhibitors of the invention are very efficient in their action on MHC class II genes expression because their target is essential for the control of MHC class II gene expression.

Thus, an important aspect of the invention relates to the identification of inhibitors of a transcription factor of the invention.

Thus, the invention includes a process for identifying inhibitors of a protein, a peptide or a nucleic acid molecule of the invention.

Potential inhibitors tested may be of natural or synthetic origin and preferably of low molecular weight.

The candidates tested may be proteins, peptides, amino acids, nucleic acids, antibodies or other molecules and may come from large collection of organic molecules which are readily available in the form of chemical libraries. Chemical libraries include combinatorial libraries or 〈〈 phage display 〉〉 libraries of peptides or antibodies.

Small molecules may be tested in large amounts using combinatorial chemistry libraries.

In a first part of this important aspect, the invention concerns inhibitors which have the capacity to inhibit a function or an activity of a transcription factor of the invention.

As indicated above, the function or activity of the molecule of interest of the invention (transcription factor of the invention) may be to allow the expression of MHC class II molecules, to allow the transcription of an MHC class II gene, to allow the expression or the translation of an MHC class II protein or peptide, to allow the formation of the RFX complex, to allow the binding of the RFX complex to the X box motif, to allow the interaction between the RFX complex and the transcription factor XZBP, NF-Y or CIITA to allow a cooperative interaction that stabilizes the higher order RFX-X2BP-NF-Y complex, to direct contacts between RFX and the co-activator CIITA, to allow binding of RFX5 to the X box, or to correct the MHC II expression defect of cell lines from patients in complementation group B.

Said inhibitors may be antibodies of the invention and especially single chain antibodies, derivatives of a protein or a peptide of the invention and especially dominant negative mutants, proteins or peptides or small molecular weight molecules.

Derivatives of a protein or a peptide of the invention are any molecules which comprise part of a protein or a peptide of the invention.

Said part of a protein or a peptide of the invention may comprise at least 6 amino acids, preferably at least 20 amino acids.

Said part may preferably comprise or consist of all or part of the Ankyrin repeat-containing region. Ankyrin repeat-containing region of humans is shown at the bottom in figure 3 (identified as ank1, ank2, ank3).

Dominant negative mutants of a protein or a peptide of the invention may be generated with known techniques as PCR mutagenesis or N or C-terminal deletion libraries. Dominant negative mutants may be generated as described in reference 51.

Antibodies of the invention are defined above .

Inhibitors of the invention may be identified or are identifiable by the following processes which are part of the invention.

The invention relates to a process for identifying inhibitors which have the capacity to inhibit a function or an activity of a transcription factor of the invention. The precise process steps may be chosen in view of the function or activity of the transcription factor of the invention which is qualitatively or quantitatively detected.

In a first embodiment, the detected function is the expression of MHC class II molecules. Then the invention relates to a process for identifying inhibitors which have the capacity to inhibit the expression of MHC class II molecules at the surface of cells or inside the cells.

If the expression of MHC class II is measured at the surface of cells, the detected function is the translation or expression of MHC II proteins or peptides.

Candidate inhibitors may then be tested for their capacity to inhibit said function in simple robust cell based assays of the expression of MHC class II molecules at the surface of cells. Such assays, based on detection with available monoclonal antibodies, are readily available. They involve both the detection of inhibition of constitutive expression of MHC class II on B lymphocyte cell lines and/or the detection of inhibition of induction of MHC class II expression by interferon gamma on any one of many inducible cell lines, such as Hela cells. These cell-based secondary screening assays can be performed on a large scale and can thus accomodate very large collections of candidate compounds.

If the expression of MHC class II is measured at the mRNA level, the detected function is the transcription of MHC class II gene.

In this case, candidate inhibitors may be tested for their capacity to inhibit said function by quantification of MHC II mRNA levels, especially by Rnase protection analysis.

Different MHC II isotypes may be searched in said quantification procedure.

In a second embodiment, the detected function is the formation of RFX complex. Then the invention relates to a process for identifying inhibitors which have the capacity to inhibit the formation of the RFX complex or the assembly of its three subunits (RFX5, RFXAP, RFXANK).

The three subunits may be mixed with the potential inhibitor in conditions which allow the formation of the RFX complex in the absence of any efficient inhibitors.

Then, the presence or absence of the RFX complex can be detected. Detection of the presence or absence of the RFX complex may be done with antibodies specific for the complete RFX complex or for the RFX complex as constituted by the 3 subunits assembled. The function of the second embodiment may be searched or measured with indirect methods as the ones used to search or measure the function in the second embodiment.

In a third embodiment, the detected function is the binding of the RFX complex to its DNA target (especially the X box motif of MHC II promoter). Then the invention relates to a process for identifying inhibitors which have the capacity to inhibit the binding of the RFX complex to its DNA target.

Said process may be a straightforward assay to measure the binding of the RFX complex (composed of RFX-ANK, RFX5 and RFXAP) to its DNA target. It may be done for example by gel retardation assays.

Said process may be performed on a large scale and will detect compounds capable of inhibiting the binding of RFX to DNA. Such an assay can be set up on a very large scale, for the screening of a large quantity of candidate molecules.

An embodiment of this process may comprise the following steps :
- the DNA fragment corresponding to the X box of the MHC II promoters is mixed with a nuclear extract of a cell and with the substance to be tested ;
- the mixture is put on a gel for running ;
- if the substance does not inhibit the formation of the RFX complex, then the RFX complex binds to the DNA and the DNA-protein association migrates slower than the non-DNA bound RFX complex.

In a fourth embodiment, the detected function is interaction between the RFX complex and at least one of the transcription factor X2BP, NF-Y and CIITA.

The invention relates to a process for identifying inhibition which have the capacity to inhibit interaction between the RFX complex and at least one of the transcription factors X2BP, NF-Y and CIITA.

Said process may comprise the mixing of the candidate inhibitor with the RFX complex and at least one of the transcription factors X2BP, NF-Y and CIITA.

Then, the presence or absence of the interaction could be measured and therefore the inhibitors identified.

In a fifth embodiment, the detected function is the correction of the MHC II expression defect of cell lines from complementation group B.

Thus, the invention relates to a process for identifying inhibitors which have the capacity to inhibit the correction by a transcription factor of the invention of MHC class II expression defect of cell lines from complementation group B.

Such a process may comprise the following steps :
- cotransfection of cells of patients from complementation group B with the potential inhibitor and a transcription factor of the invention, especially RFXANK cDNA.
- analysis of expression of HLA class II molecules.

Analysis of expression of HLA class II molecules may be done at the surface of transfectant cells (detection of peptide or protein expression) or inside the transfectant cells (detection of mRNA expression).

In a second part of this important aspect of the invention, the invention concerns inhibitors which have the capacity to inhibit the synthesis of a transcription factor of the invention.

The synthesis of the molecule of interest of the invention (transcription factor of the invention) may be the translation of the nucleic acid molecule of the invention into the protein or peptide of the invention encoded by said nucleic acid molecule.

Said inhibitors may inhibit any product which contributes to the synthesis of a transcription factor of the invention.

A product which contributes to the synthesis of a transcription factor of the invention may be a nucleic acid molecule of the invention either a DNA or a RNA molecule or a regulatory sequence of said nucleic acid molecule.

Said product may particularly be a DNA molecule coding for RFXANK as it is shown in figure 2, any DNA sequence with at least 80 % identity, preferably 90 % identity with said DNA molecule or any part of said DNA molecule or said DNA sequence.

Said product may be a RNA molecule corresponding to the DNA molecule coding for RFXANK as it is shown in figure 2, any RNA molecule with a nucleotidic sequence having at least 80 % identity, preferably 90 % identity with said RNA molecule or any part of said RNA molecules.

Said inhibitors may be a ribozyme, a DNA or a RNA antisense.

Said inhibitors may be a ribozyme, a DNA or a RNA antisense of the invention as recited above.

Complementary nucleotide sequence of the nucleic acid molecule of the invention, also referred to as 〈〈 antisense 〉〉 RNA or DNA are known to be capable of inhibiting the synthesis of the protein encoded by the revelant nucleic acid molecule of the invention.

The person skilled in the art and provided with the nucleic acid molecule sequences of the invention mentioned above will be in a position to produce and utilize the corresponding 〈〈 antisense 〉〉 RNA and DNA and to use them for the inhibition of synthesis of the transcription factor of the invention.

Inhibition of the synthesis of a transcription factor of the invention will result in the inhibition of the expression of MHC class II genes. Indeed, it is kwown from the study of mutant cells that a deficiency in protein or peptide of the invention is accompanied by a lack of expression of MHC class II genes.

The use of 〈〈 antisense 〉〉 RNA and DNA molecules, as described above, as inhibitors of MHC class II gene expression will be important in medical conditions where a reduction of MHC class II molecules is desirable, as in the case of autoimmune diseases.

The invention also relates to fragments of said nucleotide sequences, preferably to fragments of their coding regions, including fragments of complementary or 〈〈 antisense 〉〉 RNA and DNA. The person skilled in the art and provided with the sequences described above is in a position to produce the corresponding short 〈〈 antisense 〉〉 aligonucleotides and to use them to achieve inhibition of a transcription factor of the invention synthesis and therefore inhibition of MHC class II gene expression.

Said inhibitors may be produced by reference to the nucleic acid molecule of the invention and may be identified or are identifiable by a screening procedure for selecting candidate inhibitors capable of inhibiting the expression of MHC II molecules.

The screening procedure may be a test in simple robust cell based assays of the expression of MHC class II molecules at the surface of cells. Such assays, based on detection with available monoclonal antibodies, are readily available. They will involve both a search for inhibition of constitutive expression of MHC class II on B lymphocyte cell lines and/or a search for inhibition of induction of MHC class II expression by interferon gamma on any one of many inducible cell line, such as Hela cells. These cell-based secondary screening assays can be performed on a large scale and can thus accomodate very large collections of candidate compounds.

The screening procedure for selecting substances capable of inhibiting the expression of MHC II molecules may be based on the detection of the capacity to inhibit the binding of the RFX complex to its DNA target.

Said process may be a straightforward assay to measure the binding of the RFX complex (composed of RFX-ANK, RFX5 and RFXAP) to its DNA target, by gel retardation assays for example.

Said assay may be performed on a large scale and will detect compounds capable of inhibiting the binding of RFX to DNA. Such an assay can be set up on a very large scale, for the primary screening of a large quantity of candidate molecules.

This assay can be summarized in the following steps :
- the DNA fragment corresponding to the X box of the MHC II promoters is mixed with a nuclear extract of a cell and with the substance to be tested ;
- the mixture is put on a gel for running ;
- if the substance does not inhibit the formation of the RFX complex, then the RFX complex binds to the DNA and the DNA-protein association migrates slower than the non-DNA bound RFX complex.

The five embodiments of the process for identifying inhibitors which have the capacity to inhibit a function or an activity of a transcription factor of the invention may be used to identify inhibitors which have the capacity to inhibit the synthesis of a transcription factor of the invention.

Another process for identifying inhibitors of the invention is the designing of inhibitors on the basis of the three dimensional structure of the protein or peptide of the invention, an information that can be obtained from recombinant protein or peptide of the invention using state of the art technology for example X-Ray structure analysis, spectroscopic methods, etc...

The process for identifying inhibitors of the invention may in addition to the functional assays describes above, also include a preliminary or primary screening for testing a large number of candidates.

Said primary screening may be another well established procedure used on a large scale which consists in screening for the binding of molecules to a peptide or a protein of the invention (or RFX5 or RFXAP).

The peptide or protein of the invention will preferably be produced by recombinant techniques in order to obtain recombinant peptide or protein of the invention.

Such binding assays are performed on very large scales and on a routine basis by companies such as Scriptgen (Waltham, Mass) or Novalon (Durham, NC). Assays to detect such binding involve either ligand-induced change in protein conformation (Scriptgen) or ligand-induced displacement of molecules first identified as binding to the protein or peptide of the invention (Novalon).

In a preferred process for identifying inhibitors, a protein or a peptide of the invention may be used for the identification of low molecular weight inhibitor molecules as drug candidates.

Inhibitors of a protein or a peptide of the invention and of the expression of MHC class II genes, as potential drug candidates are preferably identified by a two step process :
In the first step, compatible with large scale, high throughput, screening of collections (〈〈 libraries 〉〉) of small molecular weight molecules, a protein or a peptide of the invention is used in a screening assay for molecules capable of simply binding to the protein or the peptide of the invention (= 〈〈 ligands 〉〉). Such high throughput screening assays are routinely performed by companies such as Novalon Inc. or Scriptgen Inc., and are based either on competition for binding of peptides to the target protein or on changes in protein conformation induced by binding of a ligand to the target protein. Such primary high throughput screening for high affinity ligands capable of binding to a target recombinant protein are available commercially (under contract) from such companies as Novalon or Scriptgen.
In the second step, any low molecular weight molecule identified as described above as capable of binding to a protein or a peptide of the invention, is tested in the functional RFX complex assay or in the functional MHC II expression at the surface of cells assay.

All candidate molecules are thus tested, at different concentrations, for a quantitative assessment of their anti-protein or anti-peptide of the invention inhibitory efficacy.

Substances exhibiting anti-protein or anti-peptide or anti-nucleic acid molecule of the invention inhibitory effects are then tested for obvious toxicity and pharmacokinetics assays, in order to determine if they represent valuable drug candidates.

Once a substance or a composition of substances has been identified which is capable of inhibiting a protein, a peptide or a nucleic acid molecule of the invention, its mode of action may be identified particularly its capacity to block transcription or translation of a protein or a peptide of the invention.

This capacity can be tested by carrying out a process comprising the following steps :
i) contacting the substance under test with cells expressing the protein or the peptide of the invention, as previously defined, and
ii) detecting loss of a protein or a peptide of the invention expression using a protein or a peptide of the invention or anti-protein or anti-peptide of the invention markers such as specific, labelled anti-protein or anti-peptide of the invention.

The antibodies used in such a detection process are of the type described earlier.

The invention also relates to a kit for screening substances capable of blocking a protein or a peptide of the invention activity, or of blocking transcription or translation of the protein or the peptide of the invention.

Inhibitors of the invention and especially inhibitors identifiable by the process for identifying inhibitors recited above, and compatible with cell viability, may be identified chemically and on the basis of their structure. New collections of related molecules may be generated (〈〈 analogue library 〉〉) and tested again with any one of the process for identifying inhibitors as described above. Candidate molecules capable of inhibiting any function of a transcription factor of the invention at the smallest molar concentration may be selected and considered as candidate immunosuppressive agents. They may first be tested for their effect on cells of various animal species in culture and then for in vivo studies in appropriate animal models. Such studies may test an effect on MHC class II expression, on activation of T lymphocytes in various well established experimental protocols, as well as various experimental models of organ transplantation.

An example of inhibitors of the invention may be natural mutants of RFX-ANK as present in MHC II deficiency patients of complementation group B.

Said mutants may be mutants with the DNA or amino acid sequence as shown in figure 5 or recited in the legend of figure 5 or derivates thereof. Such mutants may be splice variants.

Said mutants may be used in a DNA or RNA form. Furthermore, said DNA or RNA form could be present in a vector and especially under the control of a strong promoter, like the CMV promoter, in order to overexpress the mutants and to overcome their recessive nature.

The inhibitors of the invention are used in prophylactic and therapeutic treatment of diseases associated with aberrant or abnormal expression of MHC class II genes.

The inhibitors of the invention are useful as immunosuppressive agents, T-cell inactivation agent, anti-inflammatory agent, immunomodulators, reducing or downregulating agent of the level of a MHC II expression in a reversible or irreversible manner.

The inhibitors of the invention are useful as a pretreatment of the recipient before transplantation especially in bone-marrow transplantation to avoid or reduce the risk of rejection of the transplanted organs. The inhibitors of the invention may be administrated after transplantation as long as necessary to avoid the risk of rejection.

The invention concerns methods for treating or preventing autoimmune disease, by administering effective amounts of substances capable of downregulating the expression of HLA class II genes. Among others, these include autoimmune diseases where an aberrant and excessive expression of HLA class II molecule at the surface of certain cells is thought to be responsible for the autoimmune pathological processes. These include Insulin Dependant Diabetes (IDD), Multiple Sclerosis (MS), Lupus Erythematosis (LE) and Rheumatoid Arthritis (RA).

The invention also relates to pharmaceutical compositions comprising an inhibitor of the invention in association with physiological acceptable carriers, and to methods for the preparation of medicaments for use in therapy or prevention of diseases associated with aberrant expression of MHC class II genes using these substances.

The proteic or peptidic inhibitors of the invention can be prepared in a DNA or in a RNA form alone or with one of the known DNA or RNA formulation (liposomes, antibodies, viral vectors, ...).

A further object of the present invention is to use a protein or a peptide or a nucleic acid molecule of the invention to generate MHC class II negative transgenic animals or transgenic animals with reduced level of expression of MHC class II.

A preferred animal which can be generated as an MHC class II negative transgenic animal or as a transgenic animal with reduced level of expression of MHC class II is pig.

The generation of transgenic animals of the invention may involve the introduction of anyone of the inhibitors of the invention as a transgene.

An alternative approach consists in disrupting or replacing the RFX-ANK gene of the transgenic animal by any available technique so that this essential transcription form of the MHC class II promoter is not transcribed.

The RFX-ANK gene of the transgenic animal of interest can be identified by simple comparison with the human RFX-ANK gene. The mouse RFX-ANK gene shown in figure 3 has been identified by such comparison (see example 2).

Such an approach can be done by homologous recombination. The 〈〈 knockout 〉〉 technique may particularly be used.

The animal of the invention may then be used as a source of organs for xenogenic transplantation or as a source of cells for universal cell transplants.

The findings reported here have two other medically relevant implications. All patients with MHC-II deficiency can now be readily classified into their respective genetic complementation groups by a direct assay involving correction with each of the four regulatory genes. An assay based on the delivery of the four MHC-II regulatory genes to PBLs via an efficient retroviral vector system may be used. This assay should allow a rapid and specific genetic diagnosis that avoids the need to establish stable cell lines and to perform tedious complementation assays by cell fusion. The second implication is that the large majority of MHC-II deficiency patients can now be considered as candidates for gene therapy, as a possible alternative to bone marrow transplantation that has poor success rate in this disease³⁸. Since the three protein subunits of the RFX complex are expressed constitutively in all cell types, these three genes represent a favorable situation for genetic correction. Gene therapy is particularly relevant in the case of the RFXANK, which is affected in the large majority of patients with MHC-II deficiency. Transfer of the revelant wild-type genes into either multipotent processor cells or PBLS of MHC II expression deficiency patients may be operated.

Another object of the invention is a one-step high purification process by DNA affinity of substances comprising the following steps :
- identifying a DNA with more than one binding site ;
- adding to this DNA an extract containing the substance to be purified ;
- washing extensively (by increasing salts, or period of times, or by adding competitor DNA for example) ;
- isolating the complex by its DNA target;
- releasing the substance from its complex.

In an example, this process has allowed to obtain in a single step a yield greater than 80 % and an enrichment with respect to the acid nuclear extract of at least 3000 fold for the substance to be purified.

This new purification process is considerably better than known affinity purification procedure based on the binding of the desired substance to its DNA target. This purification procedure is better in terms of yield, enrichment, purity and simplicity.

The purification process of the invention applies to all molecules that are involved in stabilizing a DNA binding multimolecule complex and especially that are involved in stabilizing protein-protein interaction.

Thus, an efficient single step DNA affinity purification procedure that exploits the remarkable stability of a large multi-protein complex formed by RFX, X2BP, and NF-Y, which bind cooperatively to a longer segment of MHC-II promoters (the X-X2-Y box region)^{18,19,23} (see Fig. 6) has been established. The very high stability of this large complex represents an obvious advantage in terms of the yield and enrichment obtained in the purification procedure. Another major advantage is that it allows the selection of those factors that are part of a physiologically relevant multi-protein―DNA complex, at the expense of numerous other proteins capable of binding individually to the isolated X, X2, and Y motifs. Thus, in addition to RFX purification, it allows the co-purification of the biologically relevant X2 and Y box-binding factors.

A further object of the invention is a process of isolation of an unknown molecule which is involved in stabilizing a DNA binding multimolecule complex and especially that are involved in stabilizing protein-protein interaction.

Thus, here we report the isolation of a novel subunit of the RFX complex by an efficient single-step DNA-affinity purification approach. This procedure takes advantage of a strong co-operative protein-protein interaction that results in the highly stable binding of three distinct multi-protein transcription factors ― RFX, X2BP (ref.16) and NF-Y (ref.17) ― to the X-X2-Y region of MHC-II promoters^{10,18,19}. The higher order RFX―X2BP―NF-Y complex formed on DNA contains the physiologically relevant proteins involved in the activation of MHC-II promoters. With the use of this new affinity purification procedure, the novel component of the RFX complex called RFXANK was identified and the corresponding gene was isolated.

By analogy all that has been described for RFXANK is described for RFXAP provided that the amino acid or nucleotide sequence of RFXAP (reference 10) is substituted for the amino acid or nucleotide sequence of RFXANK and provided that complementation group D is substituted to complementation group B.

Thus, for example, the protein or peptide of the invention in the case of RFXAP is capable of restoring the MHC II expression in cells from MHC II deficiency patients in complementation group D and comprises all or part of the amino acid sequence shown for RFXAP in reference number 10.

This reasoning by analogy applies also to RFX5 (complementation group C) whose amino acid sequence and nucleotide sequence can be found in reference 9.

Generally, said reasoning by analogy applies to each subunit of the RFX complex.

### Figure legends

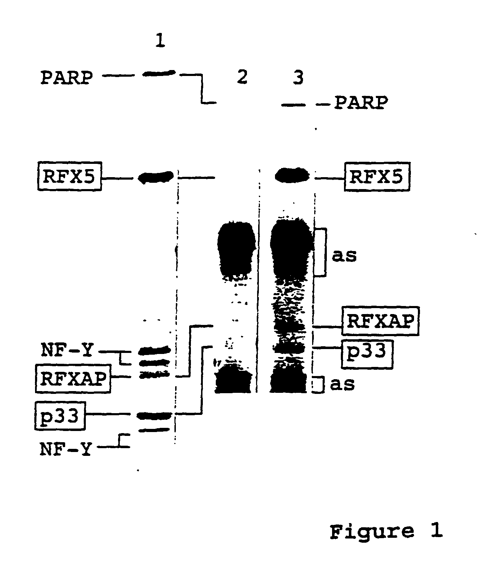

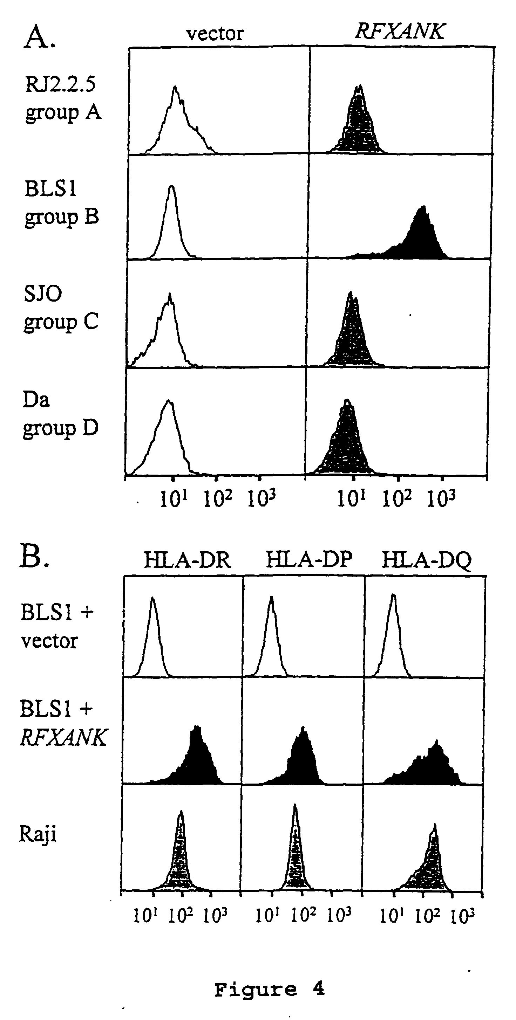

**Figure 1 :** Purification of MHC-II promoter-binding proteins and identification of a novel 33 kDa subunit of the RFX complex. Affinity purified DRA promoter binding proteins (lane 1) were immunoprecipitated with pre-immune serum (lane 2) or anti-RFXAP antibody (lane 3). Proteins were fractionated by electrophoresis in 10% SDS-polyacrylamide gels and visualised by silver-staining. The identity of the three RFX subunits (boxed: RFX5, RFXAP, and the new p33 subunit) and of other proteins (NF-Y, PARP) was determined as described in the text. Bands derived from the antiserum (as) are indicated.
**Figure 2 : Sequence analyses of RFXANK.**
   Nucleotide and amino acid sequences of RFXANK. The first nucleotides of the 10 exons that are spliced together into the RFXANK cDNA are indicated by arrows. The exon limits were established by comparing the cDNA sequence with the genomic sequence containing the entire RFXANK gene. The 'cag' trinucleotide resulting from alternative splicing of exon 4 is in lowercase and boldtype. The in-frame TGA stop codon preceding the RFXANK open reading frame is underlined. Tryptic peptides identified by microsequencing are underlined.
**Figure 3 : Sequences comparisons**
   Amino acid sequence alignment between RFXANK and homologous proteins containing ankyrin repeats. The human RFXANK sequence is shown at the top (Hs RFXANK). Identical amino acids in mouse RFXANK (Mm RFXANK) and the other proteins are shown as dashes. Gaps are represented by points. 'Hs homol' and 'Mm homol' correspond to the predicted translation products of cDNAs encoding a highly homologous protein present in humans and mice, respectively. The ankyrin repeat-containing region of mouse GABPb (ref. 28) is shown at the bottom. The secondary structure prediction of the ankyrin repeats (ank 1-3) was inferred from the known structure of GABPb (ref. 35). H, helix; T, turn
**Figure 4 :** RFXANK restores MHC-II expression in cells from patients from complementation group B.
   a, Cell lines from complementation groups A (RJ2.2.5), B (BLS1), C (SJO), and D (DA) were transfected with the empty pEBO-76PL expression vector (left) or with pEBO-RFXANK (right). Transfected cells were stained for HLA-DR expression and analyzed by FACScan.
   b, pEBO-RFXANK restores expression of all three MHC-II isotypes in BLS1 cells. BLS1 cells transfected with pEBO-76PL (upper panels) or with pEBO-RFXANK (middle panels) were analyzed by FACScan for expression of HLA-DR, -DP, and -DQ. Control Raji cells were analyzed in parallel (bottom panels).
**Figure 5 :** Mutations within the RFXANK gene in three patients in MHC-II deficiency group B.
   a, cDNA clones isolated from cells of patients BLS1, Ab, and Na lack exons 5 and 6. A schematic map of the RFXANK gene is shown at the top: non-coding exons, open bars; coding exons, filled bars. The RFXANK mRNAs isolated by RT-PCR from normal cells (wt) and from patient cells (BLS1, Ab, Na) are represented below the RFXANK gene; untranslated regions (lines), protein-coding regions (boxes), and the position of exons 4 to 7 are indicated. As a consequence of the aberrant splicing in patient cells, exons 4 and 7 of the RFXANK mRNA are joined together. This leads to a translational frame shift (amino acids in lower case) followed by a premature termination at a TGA stop codon at nucleotide 955.
   b, Short deletions affecting exon 6 of the RFXANK gene are responsible for the aberrant splicing pattern. The entire region spanning exons 4 to 7 of the RFXANK gene from patients BLS1, Na and Ab was amplified by PCR and sequenced. The sequences of splice junctions flanking exon 6 are shown: intron sequences are in lowercase and exon sequences are in uppercase. The 26 bp deletion found in patients Ab and Na (top), and the 58 bp deletion found in patient BLS1 (bottom) are boxed. The genomic region spanning exon 6 was amplified by PCR from the patients and their parents and siblings. The PCR products obtained are shown below the corresponding pedigrees. Abbreviated names of individuals are indicated; squares, males; circles, females; filled symbols - patients that developed MHC-II deficiency. Patients are homozygous for the mutated alleles (-/-), parents are heterozygous (+/-), and healthy siblings are either heterozygous (+/-), or homozygous for the wild type allele (+/+). For patient Na, the family was not available and the B cell line Raji (Ra) was used as homozygous wild type control.
**Figure 6 :** A functional RFX complex can be reconstituted with in vitro translated subunits.
   a, All three subunits are required for binding of the RFX complex to DNA. RFX5, RFXAP, and RFXANK were translated in vitro alone or in all possible combinations in the presence of [35S]-Met. Translation products were analysed by SDS-PAGE and by EMSA for their ability to bind to a X box [32P]-labeled probe. For the EMSA experiments, a crude B cell nuclear extract (NE) was used as a positive control.
   b, The RFX complex reconstituted in vitro contains all three subunits. RFX5 and RFXAP were co-translated in vitro together with RFXANK (upper panel) or a tagged version (FLAG-RFXANK) containing the FLAG epitope at its N-terminus (lower panel). The translation reactions were used for EMSA. Where indicated, binding reactions were supplemented with pre-immune serum or antibodies specific for RFX5, RFXAP or the FLAG epitope.

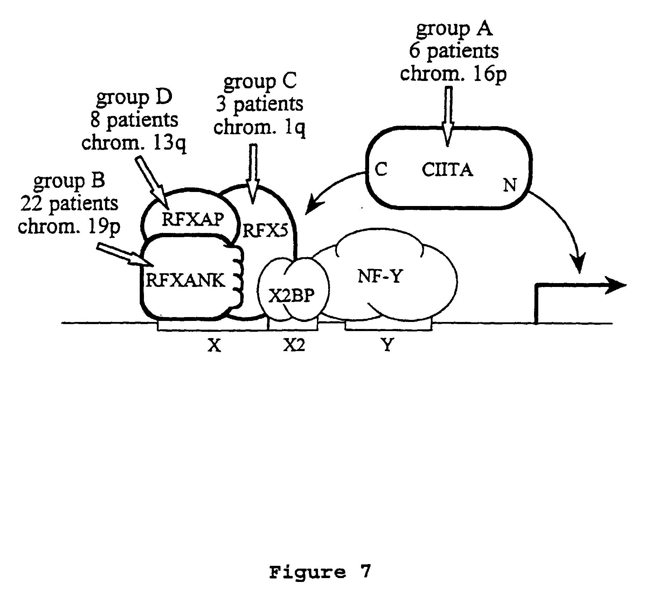

**Figure 7 :** Elucidation of the molecular defects in MHC-II deficiency has led to the identification of four essential and specific transactivators of MHC-II genes. A prototypical MHC-II promoter is depicted with conserved X, X2 and Y motifs. Each of the four transactivators affected in MHC-II deficiency is highlighted. The respective complementation groups, the number of patients reported, and the chromosomal location of each of the corresponding gene are indicated. CIITA is a highly regulated non-DNA binding co-activator that is responsible for cell-type specificity and inducibility of MHC-II expression. RFX5, RFXAP and RFXANK are the three subunits of the X box-binding RFX complex. All four genes affected in the disease are essential and specific for MHC-II gene expression. The other promoter-binding complexes, X2BP and NF-Y, are not specific for MHC-II expression and are not affected in MHC-II deficiency.

### Examples

### Example 1 : purification of the RFX complex and identification of a novel 33 kDa subunit.

Three distinct MHC-II deficiency complementation groups are characterized by a lack of binding of the RFX complex^{2,13}. Since two of these groups (C and D) result from defects in the genes encoding the RFX5 and RFXAP subunits of RFX (refs 9,10), it seemed likely that the third group (B) could be accounted for by mutations in a third subunit. The existence of a third subunit is supported by the finding that RFX5 and RFXAP are not sufficient to generate an X box specific DNA binding complex^{9,10}.

We had observed earlier that the RFX complex binds to the MHC-II promoters in a cooperative manner^{18,19,22} together with two other transcription factors, NF-Y and X2BP (refs^{16,17}). These three factors individually bind DNA with a low affinity. For example, the half life of an RFX―DNA complex is only 2 to 4 minutes^{18,19}. However, RFX, X2BP, and NF-Y combine to form an extremely stable higher order multiprotein complex on MHC-II promoters^{18,19,22}, extending the half life of the complex to more than 4 hours (ref.23). This particular feature was exploited to develop an efficient single-step DNA-affinity purification procedure.

All steps were done at 4°C. A large scale nuclear extract (1.9 g of protein) was prepared as described⁴⁴ from 2 X 10¹¹ TK6 B cells. 130 mg of extract (5 mg/ml) were supplemented with MgCl₂ to 6 mM, KCl to 100 mM, NP-40 to 0.01%, poly(dIdC) o poly(dIdC) to 0.125 mg/ml, and single-stranded E. coli DNA to 0.125 mg/ml. The extract was cleared by centrifugation and incubated with 1 mg of streptavidin magnetic beads (Dynal) coated with 30 mg of a WXX2Y DRA promoter fragment (nucleotides -150 to -47) biotinylated at the 5' end of the upper strand. Binding was allowed to proceed for 6 hours with end-over-end rotation. The beads were then washed 6 times with 40 ml buffer D (20 mM HEPES pH 7.9, 100 mM KCl, 6 mM MgCl₂, 1 mM DTT, 20% glycerol, 0.01% NP-40). Non-specific competitor DNA (salmon sperm DNA and single-stranded E. coli DNA, both at 0.125 mg/ml) was included during the last four washes. In addition, the last two washes contained 0.5 mg/ml specific competitor DNA in form of Hinf I-digested plasmid pDR300 (ref.⁴⁵). Following the washes with DNA competitors, the beads were washed a further three times with buffer D containing increased salt concentration (0.2 M KCl). The proteins that remained bound to the biotinylated DRA promoter fragment were then eluted with buffer D containing 0.6 M KCl and 10 mM EDTA instead of MgCl₂.

To summarize, a biotinylated DRA promoter fragment containing the binding sites for RFX, X2BP, and NF-Y was coupled to streptavidin-coated paramagnetic beads and incubated with a crude B cell nuclear extract to allow formation of the multiprotein complex. The beads were then washed extensively with a buffer containing non-specific competitor DNA. Finally, the degree of purification was further increased by washing the beads with a buffer containing specific competitor DNA, namely the same DRA promoter sequence that was used for selection. The rationale for including the latter step in the purification procedure was that it should permit elimination of proteins that bind the DRA promoter less stably than the RFX―X2BP― NF-Y higher order multiprotein complex. For example, other members of the X box binding protein family²⁴, such as RFX1-RFX4, would be eliminated preferentially by the final washing step because they bind to the DRA promoter with significantly less stability, and thus with a considerably shorter half-life, than the multiprotein RFX―X2BP―NF-Y complex.

This single-step affinity purification procedure turned out to be very efficient. As judged by western blotting experiments with anti-RFX5 antibodies and Bradford assays to quantitate total protein concentrations, the yield of RFX was greater than 80% and the enrichment with respect to the crude nuclear extract was at least 3000 fold. Analysis of the purified fraction by SDS-PAGE indicates that it contains only 11 different protein bands (lane 1, Fig. 1). Immunoprecipitation of the RFX complex from the affinity purified fraction was a critical step in the identification of the different protein components of this complex. Rabbit polyclonal antisera for RFX5 and RFXAP have been described previously¹⁰. Immunoprecipitation was done according to standard protocols⁴⁶. Analysis of the co-immunoprecipitated proteins was performed both by one-dimensional (lane 3, Fig. 1) and two-dimensional gel electrophoresis. Two bands were recognized by Western blot as RFX5 and RFXAP. Two additional protein bands were found at 120 kDa and 33 kDa respectively. The two proteins were isolated from a preparative gel and subjected to microsequencing by capillary liquid chromatography tandem mass spectroscopy (LC-MS/MS, ref.25).

Purified proteins were dialysed against buffer D, precipitated with acetone, and separated by SDS-PAGE. After staining the gel with Coomassie Brilliant Blue, bands were excised and subjected to microsequencing as described below.

The 120 kDa band was identified as poly(ADP-ribose) polymerase (PARP), an abundant chromatin-associated enzyme²⁶. The significance of co-immunoprecipitation of PARP with the RFX complex is not clear. On the other hand, the novel 33 kDa protein (Fig. 1), was considered a good candidate for an additional subunit of the RFX complex. This protein was reproducibly and quantitatively co-immunoprecipitated from the total affinity purified fraction with antisera directed against either RFXAP or RFX5. Its physical association with RFX5 and RFXAP is very tight because it is partially resistant to treatment with 1M Guanidine-HCl. The 33 kDa polypeptide thus behaves as a bona fide subunit of the RFX complex. From its mobility relative to RFXAP (36 kDa), the 33 kDa protein does not correspond to the band referred to as p41 by others and believed to be a component of RFX (ref.²⁷). Indeed, no additional polypeptide of this size range, besides RFXAP and the novel 33kDa protein, can be co-immunoprecipitated as part of the RFX complex.

RFXAP antiserum was immunoaffinity-purified on a column containing a C-terminal peptide of RFXAP (ref.¹⁰) coupled to CNBr-activated Sepharose (Pharmacia-Biotech). Affinity purification of RFXAP antibodies was done according to standard protocols⁴⁶.

In the DNA-affinity purified preparation (see lane 1, Fig. 1), three proteins were identified by Western blotting and immunoprecipitation as subunits of the NF-Y complex. Direct microsequencing by LC-MS/MS confirmed the presence of NF-Y peptide sequences. The remaining protein bands, distinct from both RFX and NF-Y, probably represent X2BP and/or contaminants.

### Example 2 : Isolation of the RFXANK gene.

Approximately 500 ng of the protein present in the 33 kDa band was purified by SDS-PAGE and subjected to sequence analysis by LC-MS/MS.

RFXANK was sequenced by capillary liquid chromatography tandem mass spectrometry (LC-MS/MS). The protein excised from the gel was digested with trypsin (Promega, Madison, WI) and extracted as described previously²⁵. Prior to LC-MS/MS, the peptides were vacuum dried and resuspended in H2O. Peptides were loaded onto a 75 mm RP-HPLC column, packed with 200 Å pore, 5 mm particles of Magic C18 packing material (Michrom Bioresources, Auburn, CA) at 1000 psi using a pressure bomb⁴⁷. Subsequent elution was performed at 250 nL/min after fractionation through a splitting Tee (Valco Instruments, Houston, TX) of a linear gradient that was developed for 30 min at 50 mL/min from buffer A (2 % CH3CN, 98 % H2O, 0.4 % CH3COOH, 0.005 % C4HF702) to buffer B (80 % CH3CN, 20 % H2O, 0.4 % CH3COOH, 0.005 % C4HF702) on a Michrom Ultrafast Microprotein analyzer (Michrom Bioresources, Auburn, CA). Tandem mass spectrometry was conducted on a Finnigan MAT TSQ 7000 (San Jose, CA) equipped with an in house built microspray device for peptide ionization. The instrument was run in automated mode, where parent masses were automatically selected for fragmentation⁴⁸. Collision induced dissociation (CID) spectra were correlated with database entries using the SEQUEST programme⁴⁹ and verified by manual interpretation. Databases used for CID correlation were the dbOWL http://www.biochem.ucl.ac.uk/bsm/dbbrowser/OWL/OWL.html and the
dbEST (http://www.ncbi.nlm.nih.gov/dbEST/index.html)

Perfect matches to three independent peptides (Fig. 2a) were identified in a variety of ESTs as well as in the theoretical protein product deduced from a gene identified by genomic DNA sequencing (GenBank accession number 2627294). The complete sequence of the corresponding mRNA (Fig. 2a) was determined by assembling the ESTs into a single contig and by comparing it to the genomic sequence.

Perfect matches to three RFXANK peptides were found in the putative protein product (GenBank accession number 2627294) encoded by a human gene identified by sequencing of two overlapping cosmids (GenBank accession numbers AD000812 and AC003110) derived from the short arm of chromosome 19, and in a large number of human EST clones (over 80 different ESTs; accession numbers of representative ESTs: AA282432, AA290933, AA411028, H63462, AA496321). The complete human RFXANK mRNA sequence was obtained by organizing the ESTs into a single contig.

The sequence was further confirmed experimentally by amplifying the entire open reading frame of the corresponding cDNA by RT-PCR and by sequencing several independent clones.

The resulting sequence was confirmed by comparison with the genomic sequence and by RT-PCR amplification and sequencing of RFXANK cDNA clones from control B cell lines (Raji and QBL). The following primers were used to amplify RFXANK cDNAs by PCR: 5'p33 (5'- CCGTACGCGTCTAGACCATGGAGCTTACCCAGCCTGCAGA-3'), which overlaps the translation initiation codon, and 3'p33 (5'-TTCGAATTCTCGAGTGTCTGAGTCCCCGGCA-3'), which is complementary to the 3' untranslated region of RFXANK mRNA. Homology to RFXANK mRNA is underlined. The primers contain restriction sites at their 5' ends to facilitate cloning. RFXANK cDNAs were cloned into the expression plasmid EBO-76PL (ref.⁸) and pBluescript KS (Stratagene). 12 RFXANK cDNA clones were sequenced on both strands. The nucleotide and amino acid sequences of human RFXANK were tested for homology to sequences in EMBL, GenBank, SwissProt, and dbEST. Sequence analysis was performed with PC/gene (Intelligenetics), BLAST programs available through the NCBI server (http://www.ncbi.nlm.nih.gov), and a variety of proteomics tools (http://www.expasy.ch/www/tools.html). For multiple protein sequence alignments, CLUSTALW (http://www2.ebi.ac.uk/clustalw) was used. ESTs were assembled into contigs with the TIGR Assembler (http://www.tigr.org). The search for homology to human RFXANK identified EST clones corresponding to mouse (AA435121, AA616119, AA259432, AA146531) and rat (AA851701) orthologs, and to a highly homologous gene present in both man (AA496038, AA442702, AA205305, N25678, N70046, AA418029, AA633452, H39858, R86213, AA418089, N64316, R63682, N55216) and mouse (AA245178, Z31339, AA118335). The sequences of mouse Rfxank and of the human and mouse homologues were determined by organizing the corresponding ESTs into contigs. The mouse Rfxank sequence was confined by amplifying the cDNA by RT-PCR from C57BL6 mouse spleen RNA using the following primers :
m5'p33 (5'- CCGTACGCGTCTAGACCATGGAGCCCACTCAGGTTGC -3'), which overlaps the translation initiation codon,
and m3'p33 (5'- TTCGAATTCTCGAGTGCCTGGGTTCCAGCAGG -3'), which is complementary to the 3' untranslated region of Rfxank mRNA. Homology to mouse Rfxank mRNA is underlined. The primers included 5' extensions with restriction sites that were used to clone the mouse Rfxank cDNA directly into the EBO-76PL expression vector⁸. 14 clones were sequenced on both strands.

Two splice variants were identified at approximately equal frequencies. They differ only by the insertion of a single CAG triplet (Fig. 2a) and probably result from the alternative usage of two possible splice acceptor sites situated 3 nucleotides apart upstream of exon 4. An additional minor splice variant lacking exon 5 (see Fig. 2a) was also identified, both in an EST and in one of the cDNA clones (data not shown).

The cDNA corresponding to the 33 kDa protein contains a 260 amino acid open reading frame. The translation initiation codon is preceded by an in-frame TGA stop codon, indicating that the coding region is complete. The deduced molecular weight (28.1 kDa) and isoelectric point (4.45) correspond well to the biochemical parameters determined for p33 in one- and two-dimensional gel electrophoresis (data not shown). The protein encoded by the open reading frame is novel. In particular, it exhibits no homology to either RFXAP or RFX5, the two other known subunits of the RFX complex, nor to other members of the RFX family of DNA binding proteins²⁴. A search for homology to known proteins and motifs did identify the presence of three ankyrin repeats (Fig. 2b). Together with the fact that it is an essential subunit of the RFX complex, this led us to call the protein RFXANK. Outside of the ankyrin repeat region, the only other recognizable feature is an N-terminal acidic region resembling transcription activation domains.

EST clones corresponding to mouse and rat Rfxank were also identified in the data base. Mouse ESTs were organized into a contig to generate a partial mouse sequence, which was then confirmed and completed by isolating mouse Rfxank cDNA clones by RT-PCR. Homology to human RFXANK is high (85 % overall amino acid identity), particularly within and surrounding The ankyrin repeat region (94% amino acid identity, Fig. 2b). Two different splice variants were found among mouse Rfxank cDNA clones. The major one, which is shown as the deduced amino acid sequence aligned with the human sequence in Fig. 2b, is characterized by an additional stretch of 10 amino acids that precedes the first ankyrin repeat. A minor splice variant lacking these additional 10 amino acids was also represented among the mouse cDNA clones isolated (not shown). RFXANK may belong to a family of related proteins because we identified a number of additional EST clones corresponding to at least one human and one mouse gene exhibiting a high degree of homology to RFXANK gene (Fig 2b). These are by far the most closely related sequences currently present in the data base. In addition, the ankyrin repeats of RFXANK show distinct but more limited homology (25-40% identity) to ankyrin repeat regions of a variety of other proteins^{20,21,} including the b subunit of the transcription factor GABP (ref.^{28,29}, see Fig. 2b).

### Example 3 : RFXANK restores MHC-II expression in cells from MHC-II deficiency patients in complementation group B

Cell lines and culture. The in vitro generated MHC-II negative B cell line RJ2.2.5 (ref.³⁹), the EBV transformed B cell lines from patients Ab, Na, BLS1, Da and SJO (refs.^{14,40-43}), and the control B cell lines Raji and QBL were grown in RPMI 1640 medium (GIBCO BRL) supplemented with 10% fetal calf serum, penicillin, streptomycin and glutamine. Cells were incubated at 37 oC in 5% CO2. The TK6 B cell line used for large scale nuclear extract preparation was grown at 37 oC in rollers in CO2 independent medium (GIBCO BRL) supplemented with 10% horse serum (GIBCO BRL), penicillin, streptomycin and glutamine.

It seemed likely that mutations in the newly identified gene RFXANK could account for the lack of RFX binding in the last non elucidated group of MHC-II deficiency. An RFXANK expression vector (pEBO-RFXANK) was therefore transfected into BLS1, a cell line from a patient in complementation group B. As control, pEBO-RFXANK was also transfected into cell lines from complementation groups A (RJ2.2.5), C (SJO) and D (Da), which carry mutations in CIITA, RFX5 and RFXAP, respectively^{8-10,15}.

The RFXANK cDNA (the splice variant with the additional CAG) cloned in pEBO-76PL (pEBO-RFXANK), or the empty pEBO-76PL expression vector were transfected by electroporation into RJ2.2.5, SJO, BLS1 and DA cells. Transfected cells were selected with hygromycin as described⁸. Transfected RJ2.2.5, SJO, BLS1 and DA cells were maintained under hygromycin selection for at least 10 days prior to FACScan analysis.

BLS1 transfectants were sorted for HLA-DR expression before FACScan analysis of HLA-DR, HLA-DP, HLA-DQ and MHC-I expression. Sorting was performed using an HLA-DR specific antibody and magnetic beads (Dynal) as described previously⁸. FACS analysis was performed as described¹⁰.

Expression of HLA-DR was restored in the BLS1 cell line, while no reactivation of DR expression was observed in the other three cell lines (Fig. 3a). Complementation of BLS1 with pEBO-RFXANK restored expression of all three MHC-II isotypes (HLA-DR, -DP and -DQ) to levels similar to or greater than those observed in the control B cell line Raji (Fig. 3b), indicating that all MHC-II a and b chain genes were reactivated coordinately by RFXANK. Re-expression of MHC-II by transfection with pEBO-RFXANK was also observed in Na and Ab, two other cell lines from patients in complementation group B (data not shown). In contrast to the drastic effect of RFXANK on MHC-II, no effect on cell surface expression of MHC class I (MHC-I) genes was observed in the complemented cells (data not shown).

### Example 4 : RFXANK is mutated in patients from complementation group B

The specific complementation obtained in cell lines from patients in group B suggested that RFXANK is affected in these patients. The entire coding region of RFXANK was therefore amplified by RT-PCR from the BLS1, Na and Ab cell lines, subcloned and sequenced.

The entire coding region of RFXANK mRNA was amplified by RT-PCR from patient cells using the 5'p33 and 3'p33 primers described above. PCR products were subcloned into pBluescript and sequenced on both strands. For each patient, 3 independent cDNA clones were sequenced. The genomic DNA spanning exons 4 to 7 was amplified by PCR from patient cells using an exon 4 specific primer (5'-CCAGCTCTAGACTCCACCACTCTCACCAAC-3') having a 5' extension containing an XbaI site (underlined) and an exon 7 specific primer (5'-CCTTCGAATTCTCGCTCTTTTGCCAGGATG-3') having a 5' extension containing an EcoRI site. PCR products were sublconed into pBluescript KS (Stratagene) and 6 independent subclones were sequenced for each patient. Analysis of the wild type and deleted alleles in the patients and their families was done by PCR using intronic primers flanking exon 6; (5'-GGTTCTCTAGATTGGCAGCACTGGGGATAG-3') and (5'-GCTACGAATTCCAGCAGACACAGCCAAAAC-3'). These primers carry 5' extensions containing, respectively, XbaI and EcoRI sites (underlined). The sizes of the wild type and deleted PCR products are, respectively, 265 bp, 239 bp (Ab and Na) and 207 bp (BLS1)

Analysis of several independent cDNA clones revealed the presence in all three patients of the same aberrant form of RFXANK mRNA lacking exons 5 and 6 (Fig. 4a). Splicing of exon 4 to exon 7 leads to a frame shift followed by an out of frame stop codon (Fig. 4a) and thus results in the synthesis of a severely truncated RFXANK protein lacking the entire ankyrin repeat region (see Fig. 2b).

To define the mutations leading to the aberrantly spliced RFXANK mRNA we amplified by PCR the region spanning exons 4-7 from genomic DNA isolated from patients BLS1, Na and Ab, and control individuals. The PCR products were subcloned and 6 independent subclones were sequenced for each patient. All clones from patients Na and Ab contained a 26 bp deletion that removes the splice acceptor site and the first nucleotide of exon 6; all clones from patient BLS1 contained a 58 bp deletion that removes the last 23 nucleotides and the splice donor site of exon 6 (Fig 4b).

To determine whether the patients are homozygous for the mutated alleles, the region spanning exon 6 was amplified by PCR from genomic DNA. Only the allele containing the 26 bp deletion was detected in Na and Ab, and only the allele containing the 58 bp deletion was detected in BLS1 (Fig 4b). In the case of Na, this is consistent with the fact that the parents are consanguineous⁷. For Ab and BLS1, homozygosity was established by analyzing DNA from the other family members: both parents of Ab carry the allele containing the 26 bp deletion and both parents of BLS1 carry the allele containing the 58 bp deletion (Fig 4b).

### Example 5 : RFXANK is essential for binding of the RFX complex to DNA.

Cells having mutations in RFXANK (group B), RFX5 (group C) and RFXAP (group D) all lack detectable RFX binding activity, suggesting that each of the three subunits is essential for binding. The availability of cDNA clones encoding all three subunits of the RFX complex allowed us to test this directly. The three subunits were translated in vitro, either singly or together in all possible combinations, and the translation products were tested for their ability to bind to an X box probe in an electrophoretic mobility shift assay (EMSA).

In vitro transcription-translation reactions and electrophoretic mobility shift assays (EMSA) using nuclear extracts and in vitro translated proteins were done as described^{9,22,50}. The production of polyclonal rabbit antisera specific for RFX5 and RFXAP and their use in supershift experiments have also been described¹⁰. The monoclonal anti-FLAG antibody (M2, Kodak) was used in supershift experiments at a final concentration of 20 ng/ml. The RFXANK cDNA tagged with a FLAG epitope at its N terminus was constructed as follows: The entire RFXANK open reading frame was amplified from pEBO-RFXANK plasmid by PCR with primers 3'p33 (described above)
and FLAG-5'p33 (5'-CCGTACGCGTCTAGAATGGATTACAAAGACGATGACGATAAG ATGGAGCTTACCCAGCCTGCAGAAGAC -3'). The FLAG epitope (DYKDDDDK) coding sequence is underlined. The PCR product containing the FLAG sequence fused to the 5' end of RFXANK was cloned in pBluescript KS (Stratagene).

The results demonstrate that X box-binding complexes are indeed generated, but exclusively when all three subunits are present (Fig. 5a). The RFX―DNA complexes that are generated with recombinant subunits migrate as three discrete bands, of which the lower co-migrates with the complex formed by native RFX in nuclear extracts (Fig. 5a). The reason for the aberrant mobility is not clear. Reasonable explanations include an abnormal conformation due to defective folding, abnormal posttranslational modifications, or an incorrect stoichiometry between the different subunits. Supershift experiments with antibodies directed against each of the three subunits demonstrated that the different complexes all contain RFX5, RFXAP and RFXANK (Fig. 5b). The anti-RFX5 and anti-RFXAP antibodies used in these experiments are known to supershift the RFX complex efficiently and specifically¹⁰. In the case of RFXANK, the complexes were generated with a FLAG epitope-tagged version of RFXANK, and an anti-FLAG antibody was used for the supershift. The specificity of the supershift obtained with the anti-FLAG antibody is demonstrated by the fact that migration of the complexes is not affected when they contain RFXANK lacking the FLAG tag.

### Example 6 : new single step DNA purification procedure

We had observed earlier that the RFX complex binds to the MHC-II promoters in a cooperative manner^{18,19,22} together with two other transcription factors, NF-Y and X2BP (refs^{16,17}). These three factors individually bind DNA with a low affinity. For example, the half life of an RFX―DNA complex is only 2 to 4 minutes^{18,19}. However, RFX, X2BP, and NF-Y combine to form an extremely stable higher order multiprotein complex on MHC-II promoters^{18,19,22}, extending the half life of the complex to more than 4 hours (ref.²³). This particular feature was exploited to develop an efficient single-step DNA-affinity purification procedure.

All steps were done at 4°C. A large scale nuclear extract (1.9 g of protein) was prepared as described⁴⁴ from 2 X 10¹¹ TK6 B cells. 130 mg of extract (5 mg/ml) were supplemented with MgCl₂ to 6 mM, KCl to 100 mM, NP-40 to 0.01%, poly(dIdC) o poly(dIdC) to 0.125 mg/ml, and single-stranded E. coli DNA to 0.125 mg/ml. The extract was cleared by centrifugation and incubated with 1 mg of streptavidin magnetic beads (Dynal) coated with 30 mg of a WXX2Y DRA promoter fragment (nucleotides -150 to -47) biotinylated at the 5' end of the upper strand. Binding was allowed to proceed for 6 hours with end-over-end rotation. The beads were then washed 6 times with 40 ml buffer D (20 mM HEPES pH 7.9, 100 mM KCl, 6 mM MgCl₂, 1 mM DTT, 20% glycerol, 0.01% NP-40). Non-specific competitor DNA (salmon sperm DNA and single-stranded E. coli DNA, both at 0.125 mg/ml) was included during the last four washes. In addition, the last two washes contained 0.5 mg/ml specific competitor DNA in form of Hinf I-digested plasmid pDR300 (ref.⁴⁵). Following the washes with DNA competitors, the beads were washed a further three times with buffer D containing increased salt concentration (0.2 M KCl). The proteins that remained bound to the biotinylated DRA promoter fragment were then eluted with buffer D containing 0.6 M KCl and 10 mM EDTA instead of MgCl₂.

To summarize, a biotinylated DRA promoter fragment containing the binding sites for RFX, X2BP, and NF-Y was coupled to streptavidin-coated paramagnetic beads and incubated with a crude B cell nuclear extract to allow formation of the multiprotein complex. The beads were then washed extensively with a buffer containing non-specific competitor DNA. Finally, the degree of purification was further increased by washing the beads with a buffer containing specific competitor DNA, namely the same DRA promoter sequence that was used for selection. The rationale for including the latter step in the purification procedure was that it should permit elimination of proteins that bind the DRA promoter less stably than the RFX―X2BP― NF-Y higher order multiprotein complex. For example, other members of the X box binding protein family²⁴, such as RFX1-RFX4, would be eliminated preferentially by the final washing step because they bind to the DRA promoter with significantly less stability, and thus with a considerably shorter half-life, than the multiprotein RFX―X2BP―NF-Y complex.

This single-step affinity purification procedure turned out to be very efficient. As judged by western blotting experiments with anti-RFX5 antibodies and Bradford assays to quantitate total protein concentrations, the yield of RFX was greater than 80% and an enrichment with respect to the crude nuclear extract was at least 3000 fold. Analysis of the purified fraction by SDS-PAGE indicates that it contains only 11 different protein bands (lane 1, Fig. 1). Immunoprecipitation of the RFX complex from the affinity purified fraction was a critical step in the identification of the different protein components of this complex. Rabbit polyclonal antisera for RFX5 and RFXAP have been described previously¹⁰. Immunoprecipitation was done according to standard protocols⁴⁶. Analysis of the co-immunoprecipitated proteins was performed both by one-dimensional (lane 3, Fig. 1) and two-dimensional gel electrophoresis. Two bands were recognized by Western blot as RFX5 and RFXAP. Two additional protein bands were found at 120 kDa and 33 kDa respectively. The two proteins were isolated from a preparative gel.

### References

1. de Preval, C., Lisowska-Grospierre, B., Loche, M., Griscelli, C. & Mach, B. A trans-acting class II regulatory gene unlinked to the MHC controls expression of HLA class II genes. Nature 318, 291-293 (1985).
2. Mach, B., Steimle, V., Martinez-Soria, E. & Reith, W. Regulation of MHC class II genes : Lessons from a disease. Annu.Rev.Immunol. 14, 301-331 (1996).
3. Griscelli, C., Lisowska-Grospierre, B. & Mach, B. Combined immunodeficiency with defective expression in MHC class II genes. in Immunodeficiencies (eds Rosen, F.S. & Seligman, M.) 141-154 ( Harwood Academic Publishers, Chur, Switzerland, 1993).
4. Reith, W., Steimle, V., Lisowska-Grospierre, B., Fischer, A. & Mach, B. in Molecular Basis of Major Histocompatibility Class II Deficiency. in Primary Immunodeficiency Diseases, a Molecular and Genetic Approach (eds Ochs, H., Puck, J. & Smith, E., Oxford University Press, New York, 1998).
5. Benichou, B. & Strominger, J.L. Class II-antigen-negative patient and mutant B-cell lines represent at least three, and probably four, distinct genetic defects defined by complementation analysis. Proc.Natl.Acad.Sci.USA 88, 4285-4288 (1991).
6. Seidl, C., Saraiya, C., Osterweil, Z., Fu, Y.P. & Lee, J.S. Genetic Complexity of Regulatory Mutants Defective for HLA Class- II Gene-Expression. J.Immunol. 148, 1576-1584 (1992).
7. Lisowska-Grospierre, B., Fondaneche, M.C., Rols, M.P., Griscelli, C. & Fischer, A. Two complementation groups account for most cases of inherited MHC class II deficiency. Hum.Mol.Genet. 3, 953-958 (1994).
8. Steimle, V., Otten, L.A., Zufferey, M. & Mach, B. Complementation cloning of an MHC class II transactivator mutated in hereditary MHC class II deficiency. Cell 75, 135-146 (1993).
9. Steimle, V., et al. A novel DNA binding regulatory factor is mutated in primary MHC class II deficiency (Bare Lymphocyte Syndrome). Genes & Dev. 9, 1021-1032 (1995).
10. Durand, B., et al. RFXAP, a novel subunit of the RFX DNA binding complex is mutated in MHC class II deficiency. EMBO J 16, 1045-1055 (1997).
11. Steimle, V., Siegrist, C.-A., Mottet, A., Lisowska-Grospierre, B. & Mach, B. Regulation of MHC class II expression by Interferon-gamma mediated by the transactivator gene CIITA. Science 265, 106-109 (1994).
12. Muhlethaler-Mottet, A., Otten, L.A., Steimle, V. & Mach, B. Expression of MHC class II molecules in different cellular and functional compartments is controlled by differential usage of multiple promoters of the transactivator CIITA. EMBO J. 16, 2851-2860 (1997).
13. Reith, W., et al. Congenital immunodeficiency with a regulatory defect in MHC class II gene expression lacks a specific HLA-DR promoter binding protein, RF-X. Cell 53, 897-906 (1988).
14. Herrero Sanchez, C., Reith, W., Silacci, P. & Mach, B. The DNA-binding defect observed in major histocompatibility complex class II regulatory mutants concerns only one member of a family of complexes binding to the X boxes of class II promoters. Mol.Cell Biol. 12, 4076-4083 (1992).
15. Villard, J., Lisowska-Grospierre, B., Van den Elsen, P., Fischer, A., Reith, W. & Mach, B. Mutation of RFXAP, a regulator of MHC class II genes, in primary MHC class II deficiency. N.Engl.J.Med. 337, 748-753 (1997).
16. Hasegawa, S.L. & Boss, J.M. Two B cell factors bind the HLA-DRA X box region and recognize different subsets of HLA class II promoters. Nucleic Acids Res. 19, 6269-6276 (1991).
17. Hooft Van Huijsduijnen, R., Li, X.Y., Black, D., Matthas, H., Benoist, C. & Mathis, D. Co-evolution from yeast to mouse: cDNA cloning of the two NF-Y (CP-1/CBF) subunits. EMBO J. 9, 3119-3127 (1990).
18. Reith, W., Kobr, M., Emery, P., Durand, B., Siegrist, C.A. & Mach, B. Cooperative binding between factors RFX and X2bp to the X and X2 boxes of MHC class II promoters. J.Biol.Chem. 269, 20020-20025 (1994).
19. Reith, W., Siegrist, C.A., Durand, B., Barras, E. & Mach, B. Function of major histocompatibility complex class II promoters requires cooperative binding between factors RFX and NF-Y. Proc.Natl.Acad.Sci.U.S.A. 91, 554-558 (1994).
20. Bennett, V. Ankyrins. Adaptors between diverse plasma membrane proteins and the cytoplasm. J.Biol.Chem. 267, 8703-8706 (1992).
21. Bork, P. Hundreds of ankyrin-like repeats in functionally diverse proteins: mobile modules that cross phyla horizontally? Proteins 17, 363-374 (1993).
22. Durand, B., Kobr, M., Reith, W. & Mach, B. Functional complementation of MHC class II regulatory mutants by the purified X box binding protein RFX. Mol.Cell Biol. 14, 6839-6847 (1994).
23. Louis-Plence, P., Moreno, C.S. & Boss, J.M. Formation of a regulatory factor X/X2 box-binding protein/nuclear factor-Y multiprotein complex on the conserved regulatory regions of HLA class II genes. J.Immunol. 159, 3899-3909 (1997).
24. Emery, P., Durand, B., Mach, B. & Reith, W. RFX proteins, a novel family of DNA binding proteins conserved in the eukaryotic kingdom. Nucl.Acids Res. 24, 803-807 (1996).
25. Wilm, M., et al. Femtomole sequencing of proteins from polyacrylamide gels by nanoelectrospray mass spectrometry. Nature 379, 466-469 (1996).
26. Alkhatib, H.M., et al. Cloning and expression of cDNA for human poly(ADP-ribose) polymerase [published erratum appears in Proc Natl Acad Sci U S A 1987 Jun;84(12):4088]. Proc.Natl.Acad.Sci.U.S.A. 84, 1224-1228 (1987).
27. Moreno, C.S., Rogers, E.M., Brown, J.A. & Boss, J.M. Regulatory factor X, a bare lymphocyte syndrome transcription factor, is a multimeric phosphoprotein complex. J.Immunol. 158, 5841-5848 (1997).
28. LaMarco, K., Thompson, C.C., Byers, B.P., Walton, E.M. & McKnight, S.L. Identification of Ets- and notch-related subunits in GA binding protein. Science 253, 789-792 (1991).
29. Watanabe, H., Sawada, J., Yano, K., Yamaguchi, K., Goto, M. & Handa, H. cDNA cloning of transcription factor E4TF1 subunits with Ets and notch motifs. Mol.Cell Biol. 13, 1385-1391 (1993).
30. Iwabuchi, K., Bartel, P.L., Li, B., Marraccino, R. & Fields, S. Two cellular proteins that bind to wild-type but not mutant p53. Proc.Natl.Acad.Sci.U.S.A. 91, 6098-6102 (1994).
31. Baldwin, A.S.J. The NF-kappa B and I kappa B proteins: new discoveries and insights. Annu.Rev.Immunol. 14:649-83, 649-683 (1996).
32. Baeuerle, P.A. & Baltimore, D. NF-kappa B: ten years after. Cell 87, 13-20 (1996).
33. McDonald, N.Q. & Peters, G. Ankyrin for clues about the function of p16INK4a. Nat.Struct.Biol. 5, 85-88 (1998).
34. Thompson, C.C., Brown, T.A. & McKnight, S.L. Convergence of Ets- and notch-related structural motifs in a heteromeric DNA binding complex. Science 253, 762-768 (1991).
35. Batchelor, A.H., Piper, D.E., de la Brousse, F.C., McKnight, S.L. & Wolberger, C. The structure of GABPalpha/beta: an ETS domain- ankyrin repeat heterodimer bound to DNA. Science 279, 1037-1041 (1998).
36. Latchman, D.S. Transcription-factor mutations and disease. N.Engl.J.Med. 334, 28-33 (1996).
37. Klein, C., Lisowska Grospierre, B., LeDeist, F., Fischer, A. & Griscelli, C. Major histocompatibility complex class II deficiency: clinical manifestations, immunologic features, and outcome. J.Pediatr. 123, 921-928 (1993).
38. Klein, C., et al. Bone marrow transplantation in major histocompatibility complex class II deficiency: a single-center study of 19 patients. Blood 85, 580-587 (1995).
39. Accolla, R.S. Human B cell variants immunoselected against a single Ia antigen subset have lost expression in several Ia antigen subsets. J.Exp.Med. 157, 1053-1058 (1983).
40. Lisowska-Grospierre, B., Charron, D.J., de Preval, C., Durandy, A., Griscelli, C. & Mach, B. A defect in the regulation of major histocompatibility complex class II gene expression in human HLA-DR negative lymphocytes from patients with combined immunodeficiency syndrome. J.Clin.Invest. 76, 381-385 (1985).
41. Hume, C.R., Shookster, L.A., Collins, N., O'Reilly, R. & Lee, J.S. Bare lymphocyte syndrome: altered HLA class II expression in B cell lines derived from two patients. Hum.Immunol. 25, 1-11 (1989).
42. Touraine, J.L., Betuel, H. & Souillet, G. Combined immunodeficiency disease associated with absence of cell surface HLA A and B antigen. J.Pediatr. 93, 47-51 (1978).
43. Casper, J.T., Ash, R.A., Kirchner, P., Hunter, J.B., Havens, P.L. & Chusid, M.J. Successful treatment with an unrelated-donor bone marrow transplant in an HLA-deficient patient with severe combined immune deficiency ("bare lymphocyte syndrome"). J.Pediatr. 116, 262-265 (1990).
44. Shapiro, D.J., Sharp, P.A., Wahli, W.W. & Keller, M.J. A high efficieny HELA cell nuclear transcription extract. DNA 7, 47-55 (1988).
45. Basta, P.V., Sherman, P.A. & Ting, J.P. Identification of an interferon-gamma response region 5' of the human histocompatibility leukocyte antigen DR alpha chain gene which is active in human glioblastoma multiforme lines. J Immunol. 138, 1275-1280 (1987).
46. Harlow, E. & Lane, D. Antibodies: A laboratory manual ( Cold Spring Harbor Laboratory, 1988).
47. Karleson, K.E. & Novotny, M. Separation efficiency of slurry-packed liquid chromatography microcolumns with very small inner diameters. Anal.Chem. 60, 1662-1665 (1988).
48. Yates, J.R., Eng, J.K., McCormack, A.L. & Schieltz, D. Method to correlate tandem mass spectra of modified peptides to amino acid sequences in the protein database. Anal.Chem. 67, 1426-1436 (1995).
49. Eng, J.K., McCormack, A.L. & Yates, J.R. An approach to correlate tandem mass spectral data of peptides with amino acid sequences in a protein database. J.Am.Soc.Mass Spectrom. 5, 976-989 (1994).
50. Reith, W., et al. MHC class II regulatory factor RFX has a novel DNA-binding domain and a functionally independent dimerization domain. Genes & Dev. 4, 1528-1540 (1990).
51. Bontron S. et al. Efficient Repression of Endogenous Major Histocompatibility Complex Class II Expression through Dominant Negative C II TA mutants Isolated by a Functional Selection Strategy. Molecular and Cellular Biology. 17, 4249-4258 (1997).

## Claims

1. Protein or peptide capable of restoring the MHC-II expression in cells from MHC-II deficiency patients in complementation group B and comprising all or part of the amino-acid sequence shown in figure 3.

2. Protein or peptide according to claim 1 wherein the cells are BLS 1 cell line or Na cell line or Ba cell line.

3. Protein or peptide according to claim 1 or 2 wherein the MHC-II is HLA-DR or HLA-DP or HLA-DQ.

4. Protein or peptide comprising the amino acid sequence shown in figure 2 or a part thereof, or an amino acid sequence having at least 80 % and preferably at least 90 % homology with the illustrated sequences or said part thereof.

5. Protein or peptide which is the homologous protein of a protein or a peptide of any one of claims 1 to 4 in another species than human.

6. Protein or peptide of claim 5 wherein the species is pig.

7. Antibodies capable of specifically recognising a peptide or protein according to any of claim 1 to 6.

8. Antibodies according to claim 7 which are monoclonal.

9. Antibodies according to claims 7 or 8 which are single chain antibodies.

10. Antibodies according to anyone of claims 7 to 9 which are capable of inhibiting a function or an activity of a protein or a peptide of any one of claims 1 to 6.

11. Nucleic acid molecule encoding a protein or a peptide according to any one of claims 1 to 6 or a chain of antibodies according to any one of claim 7 to 10.

12. Nucleic acid molecule according to claim 11 comprising all or part of the nucleotide sequence illustrated in figure 2.

13. Nucleic acid molecule comprising a sequence complementary to the nucleic acid molecules of any one of claims 11 to 12.

14. Nucleic acid molecule capable of hybridizing in stringent conditions, with the nucleic acid molecules of any one of claims 11 to 13.

15. Nucleic acid molecule comprising at least one of the sequences illustrated in figures 3, or a sequence exhibiting at least 90 % homology with any of these sequences, or a fragment of any of these sequences.

16. Nucleic acid molecule of anyone of claims 11 to 15 comprises all or part of the DNA molecule encoding the RFXANK gene of a species other than human.

17. Nucleic acid molecule of claim 16 wherein the species is pig.

18. Nucleic acid molecule comprising a sequence complementary to the nucleic acid molecule of anyone of claims 11, 12 or 14 to 17.

19. Anti-sense molecule or ribozyme comprising a nucleic acid molecule of claim 13 or 18.

20. Vector comprising a nucleic acid molecule of any one of claims 11 to 19.

21. Process for identifying inhibitors which have the capacity to inhibit a function or an activity of a protein or a peptide according to any one of claims 1 to 6 or of a nucleic acid molecule according to any one of claims 11 to 18 comprising detecting or measuring of said function or activity after intervention of the potential inhibitor.

22. Process according to claim 21 wherein said function or activity is the expression of MHC class II molecules.

23. Process according to claim 22 wherein the expression of MHC class II molecules is measured at the surface of cells.

24. Process according to claim 22 wherein the expression of MHC class II is measured at the mRNA level or in the cells.

25. Process according to claim 23 or 24 wherein said cells are B lymphocyte cell lines with constitutive expression of MHC class II or interferon gamma inducible cell lines.

26. Process according to claim 21 wherein said function or activity is the formation of RFX complex.

27. Process according to claim 21 wherein said function or activity is the binding of the RFX complex to its DNA target.

28. Process according to claim 27 wherein the measure or detection of the function or activity is done by gel retardation assay.

29. Process according to claim 21, wherein said function or activity is the interaction between the RFX complex and at least one of transcription factors X2BP, NF-Y and CIITA.

30. Process according to claim 21 wherein said function or activity is the correction of the MHC II expression defect of cell lines from complementation group B.

31. Process for identifying inhibitors which have the capacity to inhibit the synthesis of a protein or a peptide according to any one of claims 1 to 6 or of a nucleic acid molecule according to any one of claims 11 to 18 comprising detection or measuring a product which contributes to the synthesis of said protein or peptide after intervention of the potential inhibitor.

32. Process according to claim 31 wherein said product is mRNA.

33. Process according to any one of claims 21 to 32 comprising a preliminary screening of said potential inhibitor which consists in screening for the binding of molecules to a peptide or a protein of any one of claims 1 to 6 or nucleic acid molecule of any one of claims 11 to 18.

34. Process according to claim 33 wherein the binding of molecules is detected by ligand-induced charge in protein conformation.

35. Process according to claim 33 wherein the binding of molecules is detected by ligand-induced displacement of molecules first identified as binding to a peptide or a protein of any of claims 1 to 6.

36. Process for identifying inhibitors which have the capacity to inhibit a function, an activity or the synthesis of a protein or a peptide according to any one of claims 1 to 6 or of a nucleic acid molecule according to any one of claims 11 to 18 comprising the designing of said inhibitors on the basis of the three dimensional structure of a protein or a peptide according to any one of claims 1 to 6.

37. Process according to claim 36 wherein the three dimensional structure is obtained using X-Ray structure analysis or spectroscopic methods.

38. Inhibitors of a protein or a peptide according to any one of claims 1 to 6, or of a nucleic acid molecule according to any one of claims 11 to 18.

39. Inhibitor according to claim 38 identifiable by a process according to any one of claims 21 to 38.

40. Inhibitor according to claim 39 which is an antibody according to any one of claims 7 to 8, a nucleic acid molecule according to claim 13 or 18 a derivative of a protein or a peptide according to any one of claims 1 to 6 or of a nucleic acid molecule according to any one of claims 11 to 18, or an anti-sense molecule or a ribozyme according to claim 19.

41. Inhibitor according to claim 39 which is an antibody, a single chain antibody, a dominant negative mutant, a protein, a peptide, a small molecular weight molecule, a ribozyme or an anti-sense molecule.

42. Nucleic acid molecule encoding an inhibitor of any one of claims 38 to 41.

43. Inhibitor according to any one of claims 38 to 41 for use in therapy.

44. Pharmaceutical composition comprising an inhibitor according to any one of claims 38 to 41, optionally in association with a pharmaceutically acceptable vehicle.

45. Use of an inhibitor according to any one of claims 38 to 41 for the preparation of a medicament for use in therapy or prevention of diseases associated with aberrant expression of MHC class II genes.

46. Use of an inhibitor according to any one of claims 38 to 41 as an immunosuppressive agent
